(19) Europäisches Patentamt
European Patent Office
Office européen des brevets

(11) EP 2 453 234 B1

(12) **EUROPEAN PATENT SPECIFICATION**

(45) Date of publication and mention of the grant of the patent:
**15.01.2014 Bulletin 2014/03**

(51) Int Cl.:
***G01N 33/50*** *(2006.01)* ***C12Q 1/68*** *(2006.01)*

(21) Application number: **12154122.1**

(22) Date of filing: **29.10.2008**

(54) **Methods for prognosing the ability of a zearalenone analog compound to treat cancer**

Verfahren zur Vorhersage der Fähigkeit einer Zearalenon-Analogverbindung zur Behandlung von Krebs

Procédés pour le pronostic de la capacité d'un composé à base d'un analogue de la zéaralénone à traiter le cancer

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.10.2007 US 796 P**

(43) Date of publication of application:
**16.05.2012 Bulletin 2012/20**

(62) Document number(s) of the earlier application(s) in accordance with Art. 76 EPC:
**08845473.1 / 2 215 471**

(73) Proprietor: **Eisai R&D Management Co., Ltd.**
**Tokyo 112-8088 (JP)**

(72) Inventors:
- **Nomoto, Kenichi**
  **Belmont, MA 02478 (US)**
- **Wang, John(Yuan)**
  **Andover, MA 01810 (US)**
- **Agoulnik, Sergei**
  **Andover, MA 01810 (US)**

(74) Representative: **Cornish, Kristina Victoria Joy**
**Kilburn & Strode LLP**
**20 Red Lion Street**
**London**
**WC1R 4PJ (GB)**

(56) References cited:
**WO-A-03/076424**
**WO-A-2006/036941**
**WO-A2-2009/015368**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 453 234 B1

## Description

### BACKGROUND OF THE INVENTION

The increased number of cancer cases reported in the United States, and, indeed, around the world, is a major concern. Currently there are only a handful of treatments available for specific types of cancer, and these provide no absolute guarantee of success. In order to be most effective, these treatments require not only an early detection of the cancer, but a reliable assessment of whether the cancer can be effectively treated with known compounds, and a reliable determination of whether a cancer in a subject is sensitive to treatment,

It is known that mutations in the RAS/RAF/MEK/ERK MAPK signaling pathway are often observed in transformed cell lines and frequently linked with human cancer. Davies et al. (Nature 417:949-954, 2002), for example, identified BRAF (encoding BRAF protein, an isoform of RAF) somatic missense mutations in 67% of malignant melanomas and in 12% of colorectal cancers. Mutations in this pathway have also been associated with thyroid cancer (*e.g.*, papillary thyroid carcinoma), pancreatic cancer, brain tumors (*e.g.*, primary brain tumors), ovarian cancer, leukemia (*e.g.*, chronic myeloid leukemia and/or acute lymphoblastic leukemia (ALL)), breast cancer, neural cancer (*e.g.*, glioma, neuroblastoma or retinoblastoma), multiple myeloma, melanoma (*e.g.*, metastatic melanoma), colorectal cancer (*e.g.*, colorectal carcinoma), and B-cell lymphoma.

Recently, it was discovered that zearalenone analog compounds have unique multikinase inhibition profiles, which may be useful against specific cancers associated with mutations in the MAPK signaling pathway (see, *e.g.*, U.S. Serial No. 60/951,906, filed on July 25, 2007, and U.S. Serial No. 12/180,408, filed on July 25, 2008). Often, however, certain cancer cells are resistant to treatment with chemotherapeutic drugs. Known chemotherapeutic drugs, including zearalenone analog compounds, may not be effective for treating all known cancers. WO2006036941 relates to prognosing the sensitivity of malignant melanomas to treatment by zearalenone analogues by typing B-Raf mutations. Thus, a need exists in the art for a method of prognosing the ability of a chemotherapeutic compound such as a zearalenone analog compound to treat a cancer in a subject. Additionally, certain cancer cells may become resistant to treatment with chemotherapeutic compounds over time. Therefore, a need also exists in the art for methods of determining whether a cancer in a subject is sensitive to treatment with a chemotherapeutic compound, *e.g.*, a zearalenone analog compound.

### SUMMARY OF THE INVENTION

In its broadest sense, the invention concerns subject-matter as defined in the appended claims. The present invention provides methods for prognosing the ability of a zearalenone analog compound to treat, *e.g.*, inhibit the growth, of a cancer in a subject. The present invention is based, at least in part, on the discovery that cancer cell lines with activated MAPK signaling are sensitive to treatment with zearalenone analog compounds of formula (I).

The *BRAF* mutation (*e.g.*, V600E) was identified as useful marker for patient selection. Patients with mutated *BRAF* are predicted to respond to zearalenone analog compounds such as compound 106.

Decreases in pharmacodynamic markers such as phospho-ERK, cyclin D1 and/or phospho-pRB, or increases in CDK inhibitor, p27 provide additional surrogate markers for response to treatment with a zearalenone analog compound. Overall, these findings allow us to generate a biomarker program for zearalenone analog compounds which provides patient enrichment strategies, as well as modalities for follow-up to treatment with early assessment of drug response via pharmacodynamic monitoring.

In another aspect, the invention provides methods of prognosing the ability of a zearalenone analog compound of formula (I) to treat a cancer in a subject, the method comprising determining whether a sample derived from the subject exhibits a mutation in the *BRAF* gene, wherein the presence of a mutation in the *BRAF* gene in the sample as compared to a control sample indicates that a zearalenone analog compound has the ability to treat the cancer in the subject, thereby prognosing the ability of a zearalenone analog compound to treat the cancer in the subject.

The cancer is a *BRAF* mutated cancer. The *BRAF* mutated cancer is selected from the group consisting of metastatic melanoma, papillary thyroid carcinoma, colorectal carcinoma, and a primary brain tumor.

The cancer is selected from the group of solid tumors and hematological malignancies, including leukemias, lymphomas, and myelomas. For example, the cancer can be a cancer such as breast cancer, melanoma, ovarian cancer, thyroid cancer, pancreatic cancer, colorectal cancer, brain tumors, neural cancer, neuroblastoma, retinoblastoma, glioma, such as astrocytoma, glioblastoma multiforme or other CNS tumors, chronic lymphocytic leukemia (CLL), acute myeloid leukemia (AML), B-cell lymphomas (*e.g.*, non-Hodgkin's B-cell lymphomas), or multiple myeloma.

In one embodiment, the zearalenone analog compound is the compound:

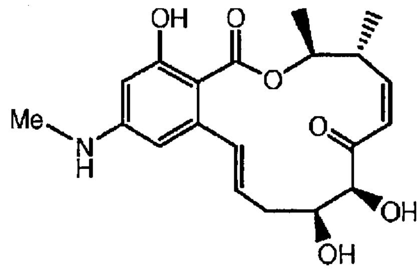

(Compound 091).

In another embodiment, the zearalenone analog compound is the compound:

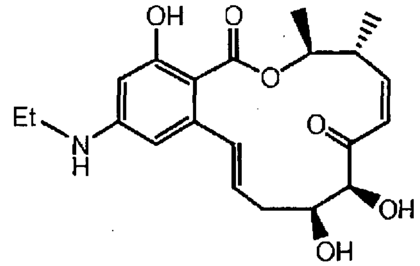

(Compound 106).

The *BRAF* gene encodes BRAF, a cytoplasmic serine/threonine kinase. Somatic mutation in the *BRAF* gene is common in human cancers. Many such mutations affect the kinase domain of the encoded protein (kinase domain mutations), and lead to elevated kinase activity of the encoded mutant BRAF protein. These mutations can lead to activation of the RAS/RAF/MEK/ERK MAPK signal transduction pathway.

In another embodiment, the mutation in the *BRAF* gene is a mutation in the kinase domain. For example, the mutation in the *BRAF* gene can be a kinase domain mutation which leads to elevated kinase activity of BRAF. In another embodiment, the mutation in the *BRAF* gene is V600E. In yet another embodiment, the mutation in the *BRAF* gene is selected from the group consisting of V600E, G464E, G464V, G466A, G466E, G466V, G469A, G469E, E586K, F595L, G596R, L597V, L597R, L597S and V600D.

In one embodiment, determining whether the sample exhibits a mutation in the *BRAF* gene is accomplished using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of the sample.

In another embodiment, determining whether the sample exhibits a mutation in the *BRAF* gene comprises measuring BRAF activity in the sample (*e.g.*, protein kinase activity of BRAF), wherein an increase in BRAF activity in the sample as compared to the control sample is an indication of a mutation in the *BRAF* gene.

The sample may be derived from a tumor biopsy.

**BRIEF DESCRIPTION OF THE FIGURES**

***Figure 1*** *is* a schematic summarizing the RAS/RAF/MEK/ERK MAPK and PI3K signaling pathways.

***Figure*** **2** is a graph demonstrating that the phosphorylation status of AKT affects the sensitivity of various cancer cell lines to compound **106**.

***Figures 3A, 3B, 3C*** and 3D are graphs and tables summarizing the cancer cell lines which were used in the experiments described herein. These tables also summarize the $IC_{50}$ values for compound **106** and compound **091** in several cancer cell lines.

***Figure 4*** is a schematic demonstrating the use of prognostic biomarkers for the enrichment of patients sensitive to compound **106** and the use of surrogate response markers to determine the response of patients after treatment with compound **106**.

***Figure 5*** is a graph demonstrating that *BRAF* mutated cancer cells produce the pro-inflammatory cytokine Interleukin-8 (IL-8).

***Figure* 6** is a graph demonstrating that IL-6 and IL-8 production is inhibited by Compound **106** in LOX melanoma cells *in vitro*.

***Figure* 7** is a graph demonstrating that tumor bearing mice sensitive to compound **106** show a significant decrease in plasma IL-8 levels after treatment with compound **106**, whereas tumor bearing mice resistant to compound **106** do not show any significant change in plasma IL-8 levels after treatment with compound **106**.

***Figure* 8** is a set of Western blots demonstrating the protein levels of several response markers, *e.g.*, phospho-ERK, Cyclin D1, p27 and phospho-pRB, after treatment with compound 106.

***Figures 9A and 9B*** are immunohistochemistry (IHC) stains demonstrating phospho-pRB and phospho-ERK staining in LOX melanoma xenografts.

***Figure 10*** is a graph demonstrating the response of s.c. DBTRG-05MG glioblastoma tumors to treatment with Compound 106.

***Figure 11*** is a graph demonstrating the response of s.c. LOX melanoma tumors to treatment with Compound 106.

## DETAILED DESCRIPTION OF THE INVENTION

The RAS/RAF/MEK/ERK MAPK signal transduction pathway regulates cell proliferation in diverse types of cells. Mutations in this pathway are often observed in transformed cell lines and frequently linked with human cancer (see, *e.g.,* Wallace et al. (2005) Current Topics in Medicinal Chemistry 5:215-219). Aspects of the present invention are based, at least in part, on the discovery that cancer cell lines with activated MAPK signaling are sensitive to treatment with a zearalenone analog compounds of formula (I), *e.g.*, Compound 106. The present invention provides methods for prognosing the ability of a zearalenone analog compound of formula (I) to treat a cancer in a subject.

In order to more clearly and concisely describe the subject matter of the claims, the following definitions are intended to provide guidance as to the meaning of specific terms used herein.

### Definitions

It is to be noted that the singular forms "a," "an," and "the" as used herein include "at least one" and "one or more" unless stated otherwise. Thus, for example, reference to "a pharmacologically acceptable carrier" may include mixtures of two or more carriers as well as a single carrier.

As used herein, the terms "prognosis", "prognose", or "prognosing" refer to a prediction of a probability, course or outcome. Specifically, "prognosing the ability of a zearalenone analog compound to treat a cancer in a subject" refers to the prediction that the zearalenone analog compound is likely to be useful for treating a cancer in a subject. For example, the prognostic methods of the instant invention provide for determining whether a sample exhibits specific characteristics (*e.g.*, activated MAPK signaling, wild-type PI3K signaling, a mutation in the *BRAF* gene, the status of AKT phosphorylation, and/or wild-type *PTEN* sequence) which can be used to predict whether a zearalenone analog compound has the ability to treat a cancer in a subject.

"Treat", "treatment,", "treating" or "treated" as used herein, refers to a cancer being cured, healed, alleviated, relieved, remedied, ameliorated, or improved. For example, the prognostic methods of the instant invention are useful in determining whether a zearalenone analog compound can slow or stop the progression of a specific cancer or a specific class of cancer (*e.g.*, a *BRAF* associated cancer).

The term "subject," as used herein, refers to animals such as mammals, including, but not limited to, humans, primates, cows, sheep, goats, horses, pigs, dogs, cats, rabbits, guinea pigs, rats, mice or other bovine, ovine, equine, canine, feline, rodent or murine species. In some embodiments, the subject is a human.

"Determining whether a sample exhibits a mutation" may be accomplished using any suitable method, such as techniques available in the art, *e.g.*, deoxyribonucleic acid sequencing of a sample, polymerase chain reaction (PCR) amplification, reverse-transcriptase PCR analysis, single-strand conformation polymorphism analysis (SSCP), mismatch

cleavage detection, heteroduplex analysis, Southern blot analysis, or Western blot analysis. These techniques are well known to one of ordinary skill in the art and are generally described in Sambrook, J. et al. (1989) "Molecular Cloning: A Laboratory Manual", Cold Spring Harbor Laboratory Press. Determining whether a sample exhibits a mutation may entail examination or all or part of a gene for the presence of a mutation (*e.g.*, in a kinase domain).

The term "mutation in the *BRAF* gene", as used herein, refers to a *BRAF* gene sequence which contains one or more mutations that lead to activation or a gain in BRAF function. BRAF mutations are well known in the art. For a review of *BRAF* mutations, see Davies et al. (2002) Nature 417:949-954 and Rodriguez-Viciana et al. (2006) Science 311:1287-1290. The most common mutation in the *BRAF* gene is referred to as the V600E mutation (originally described as V599E) and accounts for more than 90% of *BRAF* mutations. Additional mutation sites are known in the art, such as those described in the Davies *et al.* and Rodriguez-Viciana *et al.* articles cited above. For example, *BRAF* mutations include G464E, G464V, G466A, G466E, G466V, G469A, G469E, E586K, F595L, G596R, L597V, L597R, L597S and V600D.

The term *"BRAF* mutated cancer", as used herein, refers to cancers that are associated with one or more mutations in the *BRAF* gene that lead to activation or a gain in BRAF function. Human cancers often contain somatic missense mutations in the *BRAF* gene (see, *e.g.*, Davies et al. (2002) Nature 417:949). The *BRAF* mutations are often in the kinase domain of BRAF, with the predominant mutation, V600E, accounting for 90% of *BRAF* mutations. Other *BRAF* mutations include G464E, G464V, G466A, G466E, G466V, G469A, G469E, E586K, F595L, G596R, L597V, L597R, L597S and V600D. As a result of the mutation in the *BRAF* gene, *BRAF* mutated cancers demonstrate elevated kinase activity, leading to the activation of MEK (mitogen activated-protein kinase/extracellular signal-regulated kinase), which then triggers ERK phosphorylation and activates the downstream pathway. Exemplary *BRAF* mutated cancers are discussed in more detail herein, and may include, *e.g.*, melanoma (*e.g.*, metastatic melanoma), thyroid cancer (*e.g.*, papillary thyroid carcinoma), colorectal cancer (*e.g.*, colorectal carcinoma), brain tumors (*e.g.*, primary brain tumors, *e.g.*, glioblastoma), ovarian cancer, leukemia (*e.g.*, chronic myeloid leukemia and/or acute lymphoblastic leukemia (ALL)), breast cancer, neural cancer *(e.g.,* glioma, neuroblastoma or retinoblastoma), multiple myeloma, and B-cell lymphoma. Cancers designated as *"BRAF* associated" are cancers which have been chosen because of their apparent association with specific protein mutations in BRAF, as described above.

As used herein, the term "resistance" refers to the occasion where a subject becomes less responsive to a zearalenone analog compound, *e.g.*, Compound 106, over time. Accordingly, in some embodiments, resistance to a zearalenone analog compound refers to a subject's complete non-responsiveness to the compound (*e.g.*, where rate of growth of a tumor is not inhibited). Resistance to a zearalenone analog compound refers to a subject's partial non-responsiveness to the compound (*e.g.*, where the rate of growth of a tumor is inhibited only to a very low degree, such as an inhibition of the growth of a tumor by about 1%, 2%, 3%, 4%, 5%, 6%, 7%, 8%, 9%, 10%, 15%, 20% or 25%). The quality of being resistant to a zearalenone analog compound is a highly variable one, with different tumors exhibiting different levels of "resistance" to a given zearalenone analog compound, under different conditions. Resistance to a zearalenone analog compound refers to a subject's complete or partial non-responsiveness to the compound as compared to a previous administration of the compound.

The term "sensitive to treatment", as used herein, refers to the occasion where a cancer in a subject is responsive to treatment with a zearalenone analog compound, *e.g.*, Compound 106. Complete responsiveness of the cancer to a zearalenone analog compound (*e.g.*, where the growth of a tumor is inhibited) is observed or partial responsiveness of the cancer to a zearalenone analog compound (*e.g.*, where the rate of growth of a tumor is inhibited to some degree, such as an inhibition of the growth of the tumor by about 95%, 90%, 85, 80%, 75%, 70%, 65%, 60%, 55%, 50%, 45% or 40%) is observed. The quality of being sensitive to treatment with a zearalenone analog compound may vary, with different tumors exhibiting different levels of "sensitivity" to a given zearalenone analog compound, under different conditions. The term sensitive to treatment refers to the effective treatment of a cancer with a zearalenone analog compound.

The term "response marker", as used herein, refers to a gene or protein marker that is objectively measured and evaluated as an indicator that a cancer is sensitive to treatment with a zearalenone analog compound. A response marker can be a cytokine, *e.g.*, IL-8, IL-1, IL-2, IL-6 or TNF$\alpha$. A response marker can also be phospho-ERK, Cyclic D1, p27, phospho-pRB or phospho-AKT. The levels of response markers can be measured by determining the expression of the markers at the mRNA or protein level using any suitable method, such as quantitative PCR, Western blotting or ELISA techniques.

Numerous values and ranges are recited, *e.g.*, amount of a compound of the invention present in a composition. It is to be understood that where a range is given (*e.g.*, from X to Y), the range includes X, Y and all values which fall between X and Y, unless explicitly stated otherwise. The term "about" as used herein in association with parameters, ranges and amounts, means that the parameter or amount is within $\pm$ 1% of the stated parameter or amount.

*Methods of Prognosis*

The present invention provides methods for prognosing the ability of a zearalenone analog compound of formula (I) to treat cancer, such as *BRAF* mutated cancer, *e.g..* melanoma (*e.g.*, metastatic melanoma), thyroid cancer (*e.g.*, papillary thyroid carcinoma), colorectal cancer (*e.g.*, colorectal carcinoma), brain tumors (*e.g.*, primary brain tumors, glioblastoma), ovarian cancer, leukemia (*e.g.*, chronic myeloid leukemia and/or acute lymphoblastic leukemia (ALL)), breast cancer, neural cancer (*e.g.*, glioma, neuroblastoma or retinoblastoma), multiple myeloma, and B-cell lymphoma.

It is believed that the RAS/RAF/MEK/ERK MAPK signal transduction pathway (see Figure 1) regulates cell proliferation in diverse types of cells. Mutations in this pathway are often observed in transformed cell lines and frequently linked with human cancer. Davies et al. (Nature 417, 949-954, 2002) previously discovered that BRAF (encoding an isoform of RAF) somatic missense mutations occur in 67% of all malignant melanomas and 12% of all colorectal cancers. The BRAF mutants typically encode a mutation in the kinase domain of BRAF, with the predominant mutation, V600E, accounting for 90% of BRAF mutations. Other *BRAF* mutations include G464E, G464V, G466A, G466E, G466V, G469A, G469E, E586K, F595L, G596R, L597V, L597R, L597S and V600D. As a result of a mutation in the *BRAF* gene, *BRAF* mutated cancers demonstrate elevated kinase activity, leading to the activation of MEK (mitogen activated-protein kinase/ extracellular signal-regulated kinase kinase), which then triggers ERK phosphorylation and activates the downstream pathway. The invention provides a new strategy for prognosing the ability of a zearalenone analog compound of formula I to treat cancer by determining whether a sample derived from a subject with cancer exhibits a *BRAF* mutation.

Three proteins have received the most attention as targets for activated MAPK signaling (see Table 1 below, modified from Table 1 in Nature Reviews of Cancer: 4, 2004). Mutations in these three proteins could lead to activation of the MEK-ERK pathway. Specifically, ovarian cancer, thyroid cancer, colorectal cancer and melanoma show high frequencies of *BRAF* mutations.

**Table 1**

| Tumor type | Pathway mutations in patient tumors |
|---|---|
| Colon | *KRAS* (45%), *BRAF* (12%) |
| Pancreatic | *KRAS* (90%) |
| Ovarian | *BRAF* (30%) |
| Melanoma | *NRAS* (15%), *BRAF* (67%) |
| Non-small cell lung | *KRAS* (35%) |
| Papillary thyroid | *HRAS, KRAS* and *NRAS* (60%); *BRAF* (30-70%) |
| ALL, AML | *NRAS* (30%) |

For example, the mutation in the *BRAF* gene may be V600E (originally described as V599E), G464E (originally described as G463E), G464V (originally described as G465V), G466A (originally described as G465A), G466E (originally described as G465E), G466V (originally described as G465V), G469A (originally described as G468A), G469E (originally described as G468E), E586K (originally described as E585K), F595L (originally described as F594L), G596R (originally described as G595R), L597V (originally described as L596V), L597R (originally described as L596R), L597S (originally described as L596S) or V600D (originally described as V599D). As many such mutations are known, when detecting such mutations, the known wild-type sequence from normal tissues of other subjects can be considered as a control.

The mutation can be determined by any suitable method, including art known techniques, such as polymerase chain reaction (PCR) amplification, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of the gene. These techniques are well known in the art and described in, for example, Sambrook, J. et al. (1989) "Molecular Cloning: A Laboratory Manual", Cold Spring Harbor Laboratory Press.

Protein activity can be measured by any suitable method, including any of a variety of methods known in the art. For example, kinase activity can be measured by enzyme-linked immunosorbent assay (ELISA), by determining the phosphorylation state of downstream proteins using radioactive means, for example $^{32}P$ or $^{33}P$-gammaphosphate incorporation, by assays employing labeled antibodies, including immunoprecipitation, blotting, and gel electrophoresis, by immunohistochemistry, or by fluorescent in situ hybridization (FISH). Other methods for measuring kinase activity are described in WO95/04136, EP0730740B1, U.S. Patent No. 5,599,681, and U.S. Patent No. 6,942,987. For example, RAF-1 or BRAF kinase activity can be determined using RAF-1 immunoprecipitation kinase cascade kits or BRAF kinase cascade kits (Upstate Biotechnology (Millipore)), and MEK/ERK activity can be measured using a coupled MEK/ERK activation assay (see, *e.g.*, Stokoe et al. (1994) Science 264:1463-1467). ERK activity can also be determined using

immunoprecipitated protein or with a p42/p44MAP kinase enzyme assay kit (see, *e.g.*, Yoon et al. (2004) Am. J. Physiol. Renal Physiol. 286:F417-F424).

Samples useful in the methods of the invention include any tissue, cell, biopsy, bodily fluid sample, extract, fraction or component thereof, for example samples including proteins or nucleic acids. For example, a sample may be a tissue, a cell, whole blood, serum, plasma, buccal scrape, saliva, cerebrospinal fluid, urine, stool, or bronchoalveolar lavage. The tissue sample may be a gastric tissue sample, a small intestine tissue sample, a large intestine tissue sample, or a skin sample. A sample may be a tumor biopsy sample, which comprises tumor tissue from the biopsy of a tumor.

Samples may be obtained from a subject by any suitable method, including, for example, by the use of a biopsy or by scraping or swabbing an area or by using a needle to aspirate bodily fluids. Methods for collecting various samples are well known in the art. Samples derived from a subject can be obtained by such methods, and may optionally have undergone further processing steps (*e.g.*, freezing, fractionation, fixation, etc.).

Tissue samples suitable for detecting and quantitating MAPK signaling or PI3K signaling may be fresh, frozen, or fixed according to methods known to one of skill in the art. Suitable tissue samples are preferably sectioned and placed on a microscope slide for further analyses. Alternatively, solid samples, *i.e.*, tissue samples, may be solubilized and/or homogenized and subsequently analyzed as soluble extracts.

A freshly obtained biopsy sample may be frozen using, for example, liquid nitrogen or difluorodichloromethane. The frozen sample is mounted for sectioning using, for example, OCT, and serially sectioned in a cryostat. The serial sections are collected on a glass microscope slide. For immunohistochemical staining the slides may be coated with, for example, chrome-alum, gelatine or poly-L-lysine to ensure that the sections stick to the slides. Samples may be fixed and embedded prior to sectioning. For example, a tissue sample may be fixed in, for example, formalin, serially dehydrated and embedded in, for example, paraffin. Where phospho-proteins are to be detected, an inhibitor of dephosphorylation can be used in the process. For example, a reagent such as Phospho-Guard™ can be used to preserve specimens or samples for immunohistochemistry (*e.g.*, Phospho-Guard™ IHC Fixation Kit (Targeted Molecular Diagnostics)).

The skilled man can select an appropriate control sample for the assay in question. For example, a normal tissue sample may serve as a control for tumor tissue (*e.g.*, from the same subject). A sample from a subject is compared to samples obtained from normal subjects. In some cases, wild-type sequence information or the level of expression of a response marker from samples obtained from normal subjects or normal tissues can serve as controls, avoiding the need to obtain a separate control sample from the subject. For example, when determining whether a subject exhibits a mutation in the *BRAF* gene, a normal tissue sample from the same subject can be used as a control if desired. Optionally, for detection of mutations known to activate BRAF, such as V600E, G464E, G464V, G466A, G466E, G466V, G469A, G469E, E586K, F595L, G596R, L597V, L597R, L597S and V600D, the known wild-type sequence from normal tissues of other subjects can be considered as a control. In the methods of the invention, the control samples in each steps are suitable control samples. Thus, they can be from the same or different tissues and/or subjects, and can be processed in a manner suitable for assessing the mutational status, activity or response marker in question.

The expression or activity level of the BRAF protein is detected on a protein level using, for example, antibodies that specifically bind this protein, such as the ones described in, for example, U.S. Patent No. 6,982,318, U.S. Publication No. 2002/0150954, and U.S. Publication No. 2007/0020232.

<u>*Zearalenone Analog Compounds*</u>

The present invention is directed to methods for prognosing the ability of a zearalenone analog compound of formula I to treat cancer in a subject. Other Zearalenone analog compounds are well known in the art and include those disclosed in U.S. Serial No. 60/951,901, filed on July 25, 2007, 60/951,906, filed on July 25, 2007, U.S. Serial No. 12/180,408, filed on July 25, 2008, U.S. Serial No. 60/951,892, filed on July 25, 2007, U.S. Serial No. 12/180,423, filed on July 25, 2008, U.S. Patent Application No. 10/507,067, U.S. Application Publication No. 2004/0224936, GB 323845, EP606044, WO00/38674, JP840893, WO96/13259, U.S. Patent No. 5,728,726, 5,674,892, and 5,795,910. Recently, it was discovered that zearalenone analog compounds have unique multikinase inhibition profiles and can cross through the blood-brain barrier, which may be useful against specific cancers. For example, it has been shown that zearalenone analog compounds can inhibit MAPKKs, including MEK1 and MEKK1, Growth Factor Receptor Tyrosine Kinases (*e.g.*, FLT-3, TRKB, EPHA2), ABL Tyrosine Kinase, and members of the PAN-SRC tyrosine kinase family (e.g., C-src, Fyn, Lyn, Lck, Yes).

The zearalenone analog compound is a compound of formula (I):

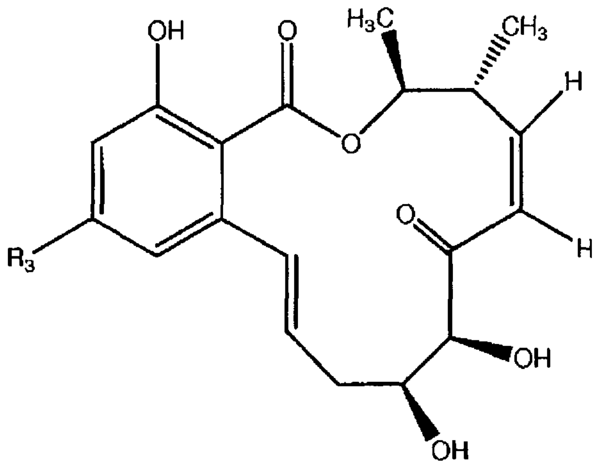

(I)

wherein

R$_3$ is -NHR$_1$, and R$_1$ is C$_1$-C$_3$ alkyl substituted with 0, 1, or 2 hydroxyl moieties, or a pharmaceutically acceptable salt or ester thereof.

In some embodiments, $R_3$ is an unsubstituted $C_{1-3}$ alkyl-amino. In some embodiments, $R_3$ is methylamino. In other embodiments, $R_3$ is ethylamino. In some embodiments, $R_3$ is a $C_{1-3}$ alkyl-amino substituted with one hydroxyl moiety. In some embodiments, $R_3$ is a $C_{1-3}$ alkyl-amino substituted with two hydroxyl moieties. The hydroxyl moieties can be on any of the carbons in the $C_{1-3}$ alkyl chain. Additionally, more than one hydroxyl moiety can be on a single carbon of the $C_{1-3}$ alkyl chain. In some embodiments, there is a hydroxyl moiety on the 2-carbon of the alkyl chain. In some embodiments, $R_3$ is hydroxyethylamino, *e.g.*, 2-hydroxyethylamino. In other embodiments, $R_3$ is dihydroxypropylamino, *e.g.*, 2,3-dihydroxypropylamino. In some embodiments, the $C_{1-3}$ alkyl is an acyclic $C_{1-3}$ alkyl chain.

As used herein, "alkyl" groups include saturated hydrocarbons having one or more carbon atoms, including straight-chain alkyl groups, cyclic alkyl groups (or "cycloalkyl" or "alicyclic" or "carbocyclic" groups) (*e.g.*, cyclopropyl), and branchedchain alkyl groups (*e.g.*, isopropyl).

In some embodiments, the zearalenone analog compound is at least one compound selected from the group consisting of compound 091 or compound 106,

| | |
|---|---|
| 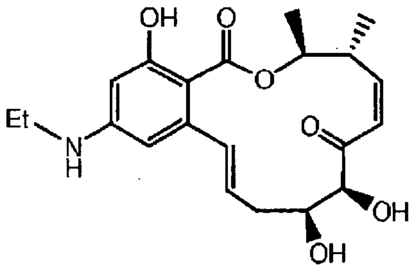 (Compound **106**) | 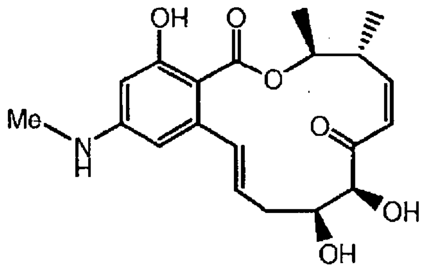 (Compound **091**) |
| 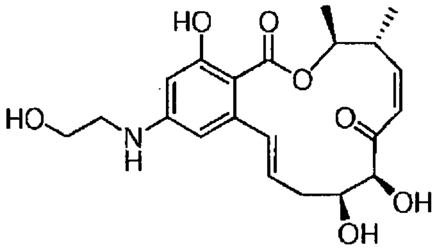 (Compound **029**) | 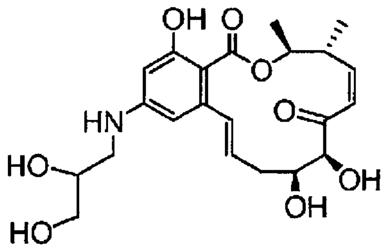 (Compound **114**) |

and pharmaceutically acceptable salts or prodrugs thereof.

A zearalenone analog compound may include one or more asymmetric centers, and, thus, can exist in various

isomeric forms, *e.g.*, stereoisomers and/or diastereomers. Thus, zearalenone analog compounds and pharmaceutical compositions containing zearalenone analog compounds may be in the form of an individual enantiomer, diastereomer or geometric isomer, or may be in the form of a mixture of stereoisomers. The zearalenone analog compounds are enantiopure compounds. Mixtures of stereoisomers or diastereomers may be used.

Zearalenone analog compounds for use in the methods may also have one or more double bonds that can exist as either the Z or E isomer, unless otherwise indicated. The specification additionally encompasses the use of compounds which exist as individual isomers substantially free of other isomers and alternatively, as mixtures of various isomers, *e.g.*, racemic mixtures of stereoisomers.

The compounds for use in the methods may further exist as one or a combination of crystalline forms, *e.g.*, polymorphs, solvates or hydrates of compound of formula (I). Various crystalline forms may be identified and/or prepared using different solvents, or different mixtures of solvents for recrystallization; by performing crystallizations at different temperatures; or by using various modes of cooling, ranging from very fast to very slow cooling during crystallizations. Different crystalline forms may also be obtained by heating or melting the compound followed by gradual or fast cooling. The presence of polymorphs may be determined by solid probe NMR spectroscopy, IR spectroscopy, differential scanning calorimetry, powder X-ray diffractogram and/or other techniques.

*Synthetic Methodology*

Zearalenone analog compounds of formula I useful for practicing the methods of the present invention may be prepared using the synthetic methods described in, for example, U.S. Serial No. 60/951,901, filed on July 25, 2007, U.S. Serial No. 60/951,906, filed on July 25, 2007, U.S. Serial No. 12/180,408, filed on July 25, 2008, U.S. Serial No. 60/951,892, filed on July 25, 2007, U.S. Serial No. 12/180,423, filed on July 25, 2008, WO 05/023792 (*e.g.*, at pages 32-38), and WO 03/076424 (*e.g.*, at pages 28-36). These references in combination with the information contained herein and the additional body of knowledge with regard to macrolide chemistry provides a person of skill in the art with guidance on synthetic strategies, protecting groups, and other materials and methods useful for the synthesis of the zearalenone analog compounds of formula I that may be used in the methods of the present invention. For example, the foregoing patent documents provide background information on preparing compounds similar to the zearalenone analog compounds of formula I described herein or relevant intermediates, as well as information on formulation, uses, and administration of such compounds.

## EXAMPLES

**Example 1: *BRAF* Mutation Cells are Sensitive to Treament with Compound 106**

Cell growth inhibition following treatment with a zearalenone analog compound, Compound 106, was assessed in a panel of cancerous cell lines from different tissue types, which carry mutations in *BRAF* and/or *RAS*. A panel of 21 cell lines was tested with varying concentrations of Compound 106. All assays were performed in a 96 well format. Cell viability was assessed after four days following treatment. Cell viability was assessed with a MTS Assay (CellTiter96®AqueousOne Solution Cell Proliferation Assay, Promega) in a panel of cell lines carrying no mutations or various *BRAF* (V600E) and/or *RAS* mutations to determine the effect of *BRAF* and *RAS* genotype on drug sensitivity.

The results are shown in Figure 3A, which is a graph depicting the $IC_{50}$ values for the cell growth inhibition of several cell lines and demonstrates that *BRAF* mutated colorectal cancer, breast cancer and melanoma cancer cell lines were all sensitive to treatment with Compound 106. *BRAF* mutated cell lines, such as the colorectal cancer cell lines HT-29 and Colo-205, breast cancer cell lines MDA-MB-435 and DU4475, and melanoma cell lines SK-MEL-3, SK-MEL-24 and SK-MEL-28 were very sensitive to treatment with the zearalenone analog Compound 106 and had an $IC_{50}$ value of less than 100 nM. These results demonstrate that cell lines with a *BRAF* mutation are more sensitive to treatment with a zearalenone analog compound, *e.g.*, Compound 106.

In a separate experiment, Compounds 091 and 106 were tested in solid cancer cell lines from different tissue types (shown in Figure 3B for 21 cell lines for compound 091 and 19 cell lines for compound 106). All cell lines were introduced into 96-well plates and grown in the absence or continuous presence of 0.3-10000 nM of either compound 091 or compound 106 for 96 hours. Cell growth was assessed using a CellTiter-Glo® Luminescent Cell Viability Assay (Promega) or a methylene blue assay. $IC_{50}$ values were determined as the concentration of a substance which inhibits cell growth by 50% compared to untreated cell populations. Cell lines which carried a *BRAF* V600E mutation were very sensitive to Compounds 091 and 106 in the low-nM or sub-μM concentration range, as shown in Figure 3B.

Cell viability was also assessed in a panel of 31 melanoma cell lines carrying different mutations in *BRAF* following treatment with a zearalenone analog compound, Compound 106. All assays were performed in a 96 well format. Cell viability was assessed after four days following treatment. Cell viability was assessed with a MTS Assay (CellTiter96®AqueousOne Solution Cell Proliferation Assay, Promega) in a panel of cell lines carrying various *BRAF*

mutations to determine the effect of *BRAF* genotype on drug sensitivity. The mutational status of these cell lines and IC50 in nmol/L is depicted in Figure 3C.

An analysis of the results is summarized in Table 2, which illustrates that cell lines carrying mutations in *BRAF* were statistically associated with sensitivity to Compound 106. Sensitivity was defined as an $IC_{50}$ < 100nmoL. Specifically, of the 20 cell lines carrying mutations in *BRAF* (20 cell lines), 75% of the cell lines were sensitive to Compound 106. Conversely, in the 11 cell lines with wild-type *BRAF*, only 27% of the cell lines were sensitive to Compound 106 (Fisher's Exact test, two tailed =P<0.01).

**Table 2:** Effect of *BRAF* Mutation on Compound 106 Sensitivity in a Panel of 31 Melanoma Cell Lines

| **Cut-Off $IC_{50}$<100 nmol/L** | **Cell Lines with Wild-Type *BRAF* Gene (11 cell lines)** | **Cell Lines with Mutant *BRAF* Gene (20 cell lines)** | **Total Cell Lines (31 cell lines)** |
|---|---|---|---|
| % of Sensitive | **3/11 (27%)** | **15/20 (75%)** | 18/31 (58%) |
| % of Less Sensitive | 8/11 (73%) | 5/20 (25%) | 13/31 (42%) |
| Total | 1.1/11 (100%) | 20/20 (100%) | 31/31 (100%) |

**<u>Example 2: Effect of *BRAF* and *PTEN* Mutations and *BRAF* and phospho-AKT Levels on Compound 106 Sensitivity in a Panel of Melanoma Cell Lines</u>**

Cell viability was assessed in a panel of melanoma cell lines carrying different mutations in *BRAF*, and *PTEN* following treatment with the MEK inhibitor, Compound 106. The panel of 31 melanoma cell lines was tested with eight concentrations of Compound 106 ranging from 10μmol/L to 0.0003μmol/L of Compound 106. (The panel of cell lines also carries mutations in one or more additional genes associated with cancer).

All assays were performed in a 96 well format. Cell viability was assessed after four days following treatment. Cell viability was assessed with a MTS Assay (CellTiter96®AqueousOne Solution Cell Proliferation Assay, Promega) to determine the effect of genotype on drug sensitivity. Individual $IC_{50}$ values are shown in Figure 3C.

Further analysis of the results was conducted in which sensitivity was defined as an $IC_{50}$ < 500nmol/L. The results of this analysis are summarized in Table 3 and reveal that sensitivity to Compound 106 was statistically associated with wild-type *PTEN* status. Specifically, of the 23 cell lines with wild-type *PTEN,* 12 cell lines were sensitive to Compound 106. Conversely, in the 8 cell lines with mutant *PTEN* or loss of *PTEN*, only 3 cell lines were sensitive (Fisher's Exact test, two tailed = P<0.01).

Phospho-AKT expression levels were also assessed. Table 3 illustrates that phospho-AKT expression affects sensitivity to Compound 106.

**Table 3:** *PTEN* Mutation/Deletion or p-AKT Levels Modulate Sensitivity to Compound 106 in 20 Melanoma Cell Lines Carrying *BRAF* Mutations

| | % of Cell lines with $IC_{50}$ <100 nmol/L | *BRAF* Wild Type (11 cell lines) | *BRAF* Mutant (20 cell lines) | Total |
|---|---|---|---|---|
| *PTEN* gene | Wild Type (23 cell lines) | 3/9 (33%) | **12/14 (86%)** | 15/23 (65%) |
| | Mutant (8 cell lines) | 0/2 (0%) | **3/6 (50%)** | 4/8 (50%) |
| | Total | 3/11 (27%) | 15/20 (75%) | **19/31 (61%)** |
| p-AKT expression | Low (G0-G1) | **3/10 (30%)** | **11/14 (79%)** | 14/24 (58%) |
| | High (G2-G3) | **0/1 (0%)** | **4/6 (67%)** | 4/7 (57%) |
| | Total | 3/11 (27%) | 15/20 (75%) | **18/31 (58%)** |

In a separate experiment, Compound 106 was tested in a panel of *BRAF* mutant (V600E) melanoma cell lines. Some of the cell lines contained mutations in the *PTEN* gene, as shown in Figure 3D. (This set of cell lines represents 17 additional cell lines relative to Figure 3C.) The melanoma cell lines were introduced into 96-well plates and grown in the absence or continuous presence of compound 106. $IC_{50}$ values were determined as the concentration of a substance which inhibits cell growth by 50% compared to untreated cell populations. Cell lines which carried a *BRAF* mutation and

wild-type *PTEN* status were very sensitive to Compound 106, as shown in Figure 3D.

These results demonstrate that cell lines with activated MAPK signaling and wild-type PI3K signaling are more sensitive to a zearalenone analog compound, *e.g.*, Compound 106.

**Example 3: AKT Phosphorylation Status Affects Compound 106 Sensitivity in a Panel of Melanoma Cell Lines**

In order to determine whether Compound 106 resistance is associated with constitutive AKT phosphorylation as a result of activation of the PI3K signaling pathway, a panel of 10 *BRAF* mutated melanoma cell lines and a *BRAF* wild-type glioblastoma cancer cell line, SF-295, were tested using different concentrations of Compound 106 ranging from 10μmol/L to 0.0003μmol/L.

All assays were performed in a 96 well format. For these cell lines, protein lysates were collected in a separate experiment, which allowed for the evaluation of the constitutive level of AKT phosphorylation and correlation between the level of phosphorylated AKT (pAKT) and Compound 106 sensitivity. Cell growth was assessed using CellTiter-Glo® Luminescent Cell Viability Assay (Promega). Phosphorylated AKT protein was analyzed by Western blotting, with total AKT being used as a loading control.

Elevated expression of pAKT, which can reflect activation of the PI3K signaling pathway, was observed in cell lines which are resistant to Compound 106, including RPMI-7951 and SF-295 (see, *e.g.*, Figure 2). Elevated expression of pAKT was not observed in cell lines which are sensitive to Compound 106. These data demonstrate that a zearalenone analog compound, *e.g.*, Compound 106, has the ability to treat cancer in cell lines with activated MAPK signaling and wild-type PI3K signaling (see, *e.g.*, Figure 4). These data further demonstrate that, a zearalenone analog compound, *e.g.*, Compound 106, has the ability to treat cancer in cancer cell lines containing a mutated *BRAF* and a wild-type *PTEN.* Taken together, these techniques provide a method of prognosing the ability of a zearalenone analog compound to treat cancer in a subject.

**Example 4: *BRAF* Mutated Cancer Cells Produce Pro-Inflammatory Cytokine Interleukin-8 (IL-8)**

In order to determine whether IL-8 is produced by melanoma cells, fourteen melanoma cell lines carrying the V600E *BRAF* mutation were evaluated *in vitro* for IL-8 cytokine expression levels by ELISA. As demonstrated in Figure 5, LOX, SK-MEL-24, UACC-62, and COLO-829 cell lines, which carry a *BRAF* mutation, all showed high IL-8 expression at the protein level.

**Example 5: Change in Plasma IL-8 in Tumor Bearing Mice Treated with Compound 106**

In order to determine whether plasma IL-8 levels could be measurable in tumor xenograft bearing mice and whether plasma IL-8 levels change after treatment with Compound 106, COLO-829 and UACC-62 melanoma xenografts were established as Compound 106 sensitive xenografts in female nude mice. SF-295 human glioblastoma (BRAF wild-type) xenografts were established as an Compound 106 resistant tumor model in female nude mice. Female athymic NU/NU mice were inoculated subcutaneously with COLO-829, UACC-62 human melanoma cancer cells, or SF-295 human glioblastoma cells. Those animals that developed tumors of approximately 250 $mm^3$ were selected and randomized to two groups. The experiment consisted of a vehicle-treated and 40 mg/kg Compound 106-treated groups of 8 mice per group on the first day of treatment. Compound 106 was administered intravenously (i.v.) on a QD x 4 (every day for a total of four injections) dosing schedule. The subcutaneous tumor volumes were measured on the first day of treatment and 24 hours after the fourth treatment. Heparinized blood was collected from these mice 24 hours after the fourth treatment with Compound 106 using a cardiac puncture, and plasma was isolated. The levels of human IL-8 in the plasma were determined by ELISA.

As demonstrated in Figure 7, at 40 mg/kg, Compound 106 almost completely blocked plasma IL-8 levels 24 hours after the fourth dosing in the xenografts which were sensitive to Compound 106, namely UACC-62 and COLO-829. In contrast, treatments with Compound 106 did not change the plasma IL-8 levels in SF-295 tumor bearing mice, which were resistant to treatment with Compound 106. Based on the foregoing results, it is evident that a reduction in plasma IL-8 may serve as a surrogate marker to measure the response of a tumor to a zearalenone analog compound, *e.g.*, Compound 106.

**Example 6: Plasma IL-8 Levels can be used to Detect a Response to Compound 106**

In order to determine whether plasma IL-8 can be detected in plasma, or blood, from human cancer patients, six plasma samples from melanoma patients were obtained from a tumor tissue bank (Asterand). Six melanoma tissues from metastatic sites were also obtained from the same patients. As a control, six plasma samples were also obtained from healthy volunteers. IL-8 plasma levels were determined by ELISA. *BRAF* mutations were detected by PCR analysis.

As shown in Table 4, IL-8 was detected in all six clinical samples tested. All six cancer patients had a *BRAF* mutation, as indicated by the PCR analysis.

**Table 4:** Plasma IL-8 Levels in Advanced Melanoma Patients

| Sample ID | Sex | Tissue | *BRAF* Mutation (indicated by the bolded, underlined nucleotide) | Plasma IL-8 (pg/mL) |
|---|---|---|---|---|
| 30517 | Female | Lymph Node | **A**GG | 26.5 |
| 30529 | Male | Soft Tissue | G**A**G | 12.0 |
| 31068 | Female | Lymph Node | A**A**G | 13.4 |
| 40968 | Male | Small Intestine | G**A**G | 48.2 |
| 48323 | Female | Lymph Node | G**A**G | 41.7 |
| 48617 | Female | Lymph Node | G**A**G | 155.0 |
| Normal | | | GTG | <3 |
| <3; below detection limit | | | | |

These results demonstrate that IL-8 can be detected in plasma from patients with advanced stage melanoma.

**Example 7: Zearalenone Analog Compounds Decrease Protein Levels of IL-8 and IL-6 in Cancer Cell Lines and Affect Secretion of IL-8 and IL-6 by *BRAF* Mutated Cells**

The purpose of this study was to investigate the effects of a zearalenone analog compound, such as Compound 106, *in vitro* on the secretion of IL-8 and IL-6 by *BRAF*-mutated LOX melanoma cells (Davies H. et al., "Mutations of the BRAF gene in human cancer", Nature, 417: 949-954 (2002)).

Compound 106 (5.0 mg) was weighed and dissolved in 100% anhydrous DMSO (dimethyl sulfoxide, Sigma-Aldrich®, St. Louis, MO) to produce a 10 mmol/L stock solution of 3.89 mg/mL. Aliquots of the 10 mmol/L stock solution were stored at-80°C. Then, on each day of an experiment, an aliquot of the stock solution was thawed and diluted 1:10 by adding RPMI-1640 culture medium to obtain a 1 mmol/L solution. Four serial 1:10 dilutions were made from 1 mmol/L working solution by adding 10% DMSO in RPMI-1640 culture medium to obtain 4 additional working solutions. Each of these working stock solutions was further diluted with culture media to obtain diluted working solutions ranging from 1 nmol/L to 10,000 nmol/L. Five mL of each of these diluted working solutions was added to each dish.

LOX human melanoma cells were originally obtained from the DCTD Tumor Repository (Frederick, MD). The cells were grown in monolayer cultures in RPMI-1640 growth media containing 10% fetal bovine serum (FBS) at 37°C in a 5% $CO_2$ humidified incubator.

LOX human melanoma cells were plated at 1 x $10^6$ cells into 100 mm dishes. After 48 hours, cells were washed three times with phosphate buffered saline (PBS) and were cultured with RPMI-1640 medium containing 0.1% FBS for another 24 hours. After removing the cell culture medium, fresh RPMI-1640 medium containing Compound 106 (1, 10, 100, 1000, or 10,000 nmol/L) was added to each culture dish and incubated for 24 hours (see Table 5). The control received 0.1% DMSO in RPMI-1640 culture medium alone to measure spontaneous secretion of IL-6 and IL-8. Three separate experiments were performed in duplicate to calculate the mean ± SEM.

**Table 5:** Sample Combinations for Determining IL-6 and IL-8 Secretion

| Sample | Cell number | Treatment |
|---|---|---|
| 1 | No cells (blank) | 0.1% DMSO in culture medium (vehicle) |
| 2 | No cells (blank) | 0.1% DMSO in culture medium (vehicle) |
| 3 | 1 x $10^6$ | 0.1% DMSO in culture medium (vehicle) |
| 4 | 1 x $10^6$ | 0.1% DMSO in culture medium (vehicle) |
| 5 | 1 x $10^6$ | 1 nmoL Compound 106 |
| 6 | 1 x $10^6$ | 1 nmoL Compound 106 |
| 7 | 1 x $10^6$ | 10 nmoL Compound 106 |

(continued)

| Sample | Cell number | Treatment |
|---|---|---|
| 8 | 1 x $10^6$ | 10 nmoL Compound 106 |
| 9 | 1 x $10^6$ | 100 nmoL Compound 106 |
| 10 | 1 x $10^6$ | 100 nmoL Compound 106 |
| 11 | 1 x $10^6$ | 1,000 nmoL Compound 106 |
| 12 | 1 x $10^6$ | 1,000 nmoL Compound 106 |
| 13 | 1 x $10^6$ | 10,000 nmoL Compound 106 |
| 14 | 1 x $10^6$ | 10;000 nmoL Compound 106 |

After a 24 hour incubation with Compound 106 or 0.1% DMSO, 5 mL of cell culture supernatant was collected. Any particulate material was removed by centrifugation. All samples were stored in the -80°C freezer until analysis. Human IL-6 or IL-8 was determined using an ELISA kit (Human IL-8 ELISA Set, Catalog No. 555244, BD Biosciences, San Diego, CA and Human IL-6 ELISA Set, Catalog No. 555220, BD Biosciences, San Diego, CA) with the assay procedure provided by the kits. Absorbance was read on a VERSAMAX™ (Molecular Devices, now part of MDS Analytical Technologies, Sunnyvale CA).

After removing the cell culture supernatant, 1.0 mL of trypsin was added to the cells for 3 minutes, and then 4 mL of media containing 10% FBS was added for cell counting. Cells were counted using a hematocytometer (Bright Line Counting Chamber, Hausser Scientific, Horsham, PA) with a depth of 0.1 mm. The total number of cells was calculated as follows:

$$\text{Total number of cells in 5 mL} = C \times 10^4 \text{ /mL} \times 5\text{mL (C: actual cell count in 1 mm}^2\text{)}$$

The level of IL-6 or IL-8 was determined as ng/1 x $10^6$ cells. Inhibition of IL-6 or IL-8 secretion by Compound 106 was expressed as percent of control using the formula below:

$$\text{Percent of control} = \text{(value of treatment} - \text{mean value of blank)/(value of control} - \text{mean value of blank)} \times 100$$

The software, Graphpad Prism® (ver. 4, San Diego, CA) was used for calculation of mean $IC_{50}$ values.

LOX human melanoma cells, which carry a *BRAF* mutation, spontaneously secreted IL-6 (15.5 ± 5.1 ng/million cells) and IL-8 (104.7 ± 38.9 ng/million cells) up to 24 hours, respectively (Table 6 and Table 7). The protein levels of IL-6 and IL-8 were decreased by Compound 106 in the LOX melanoma cell line with mean calculated $IC_{50}$ values of 21.8 and 10.5 nmol/L, respectively, in a concentration dependent manner (Figure 6 and Tables 8 and 9). These results demonstrate that changes in levels of these proteins can serve as pharmacodynamic markers to measure biological responses to a zearalenone analog compound, such as Compound 106.

**Table 6:** Inhibitory Effect of Compound 106 on Spontaneous IL-6 Secretion in Three Separate Experiments

| Compound 106 (nmol/L) | IL-6 (pg/million cells) | | | IL-6 (ng/million cells) | |
|---|---|---|---|---|---|
| | Experiment 1 | Experiment 2 | Experiment 3 | Mean | SEM |
| 0 | 11998 | 25610 | 8905 | 15.5 | 5.1 |
| 1 | 5890 | 13741 | 10140 | 9.9 | 2.3 |
| 10 | 3253 | 12105 | 10745 | 8.7 | 2.8 |
| 100 | 347 | 643 | 485 | 0.5 | 0.1 |
| 1.000 | 547 | 986 | 257 | 0.6 | 0.2 |
| 10.000 | 165 | 337 | 132 | 0.2 | 0.1 |

**Table 7:** Inhibitory Effect of Compound 106 on Spontaneous IL-8 Secretion m Three Separate Experiments

| Compound 106 (nmol/L) | IL-8 (pg/million cells) | | | IL-8 (ng/million cells) | |
|---|---|---|---|---|---|
| | Experiment 1 | Experiment 2 | Experiment 3 | Mean | SEM |
| 0 | 105021 | 172037 | 37172 | 104.7 | 38.9 |
| 1 | 112318 | 213228 | 60753 | 128.8 | 44.8 |
| 10 | 47049 | 90632 | 42942 | 60.2 | 15.3 |
| 100 | 3079 | 3249 | 3101 | 3.1 | 0.1 |
| 1,000 | 3390 | 2273 | 1494 | 2.4 | 0.6 |
| 10,000 | 5179 | 1384 | 2179 | 2.9 | 12 |

**Table 8:** Inhibitory Effect of Compound 106 on Protein Level of IL-6 in Three Separate Experiments

| Compound 106 (nmol/L) | Percent (%) of control | | | Mean % of control | SEM | n |
|---|---|---|---|---|---|---|
| | Experiment 1 | Experiment 2 | Experiment 3 | | | |
| 0.0 | 100 | 100 | 100 | 100 | 0 | 3 |
| 1 | 461 | 53.6 | 115.0 | 722 | 20.9 | 3 |
| 10 | 21.5 | 47.1 | 121.3 | 65.0 | 28.4 | 3 |
| 100 | 29 | 2.5 | 5.5 | 3.6 | 0.9 | 3 |
| 1,000 | 46 | 3 8 | 2.9 | 3.8 | 0.5 | 3 |
| 10,000 | 1.3 | 1.3 | 1.6 | 1.4 | 0.0 | 3 |

**Table 9:** Inhibitory Effect of Compound 106 on Protein Level of IL-8 in Three Separate Experiments

| Compound 106 (nmol/L) | Percent (%) of control | | | Mean % of control | SEM | n |
|---|---|---|---|---|---|---|
| | Experiment 1 | Experiment 2 | Experiment 3 | | | |
| 0.0 | 100 | 100 | 100 | 100 | 0 | 3 |
| 1 | 106.9 | 123.9 | 163.4 | 131.4 | 16.7 | 3 |
| 10 | 44.8 | 52.7 | 115.5 | 71.0 | 22.4 | 3 |
| 100 | 29 | 1.9 | 8.3 | 4.4 | 20 | 3 |
| 1,000 | 3.2 | 1.3 | 4.0 | 29 | 0.8 | 3 |
| 10,000 | 4.9 | 0.8 | 5.9 | 3.9 | 1,6 | 3 |

Moreover, as demonstrated in Figure 6, treatment with Compound 106 almost completely abrogated IL-6 ($IC_{50}$ of 21.8 nmol/L) and IL-8 ($IC_{50}$ of 10.5 nmol/L) protein expression in the LOX melanoma cell line, which cames the V600E *BRAF* mutation and is wild type for *PTEN.* Based on the foregoing results, it is evident that a reduction in plasma IL-8 and/or IL-6 levels serves as a surrogate marker to measure the response of a tumor to a zearalenone analog compound, *e.g.*, Compound 106.

**Example 8: Levels of IL-8 and IL-6 Indicate Whether a Cancer is Sensitive to Treatment with a Zearalenone Analog Compound**

In order to determine whether a cancer is sensitive to treatment with a zearalenone analog compound, the level of IL-6 or IL-8 is evaluated at various times in plasma using an ELISA assay. Blood is drawn prior to treatment, during treatment and after treatment. For example, blood can be drawn prior to infusion, just before the end of infusion, 8, 24, 48 and 72 hours after the end of infusion. This can be done for one or more infusions of a treatment cycle.

In addition, quantitative PCR is performed on tumor biopsy tissue obtained prior to treatment and post-infusion to determine mRNA levels of IL-6 and/or IL-8.

In each case above, the level of IL-6 or the level of IL-8 is used as a pharmacodynamic marker to assess the effect of zearalenone analog compound (as a surrogate marker of drug efficacy). A decrease in IL-6 or IL-8 levels during or post-treatment indicates that the cancer is sensitive to treatment with a zearalenone analog compound.

**Example 9: Protein Levels of Response Markers After Treatment with Compound 106**

In order to identify markers that could serve as response markers for determining whether a cancer in a subject is sensitive to treatment with a zearalenone analog compound, phospho-ERK, total ERK protein, phospho-pRB, total pRB, and Cyclin D1 protein levels in a cell line sensitive to Compound 106 (*e.g.*, SK-MEL-28) and cell lines resistant to Compound 106 (*e.g.*, AU-565) were examined by Western blotting. 2 x $10^6$ cancer cells (SK-MEL-28 and AU-565) were plated into a 100 mm dish and incubated for two days. Test compound, *e.g.*, Compound 106, was then added to each dish for 24 hours at a concentration ranging from 10 nmol/L to 3 $\mu$mol/L. Culture medium was added to the cells as a control. SK-MEL-28 and AU-565 cell lysate proteins were subjected to SDS-PAGE under reducing condition and immunoblotted with an antibody against phospho-ERK1/2 (catalog #9101, Cell Signaling Technology®, Danvers, MA), phospho-pRB (catalog #9307, Cell Signaling Technology®, Danvers, MA), or Cyclin D1 (catalog# sc-8396, Santa Cruz, CA). ERK1 (catalog# sc-93, Santa Cruz, CA) or pRb (catalog # sc-102, Santa Cruz, CA) antibody was used for the detection of the total amount of protein.

Expression levels of ERK and proteins which are important for the $G_1$/S transition (Cyclin D1, p27, phospho-pRB) were examined by Western blotting in the sensitive cell line, SK-MEL-28, and in the resistant cell line, AU-565. As indicated in Figure 8, protein levels of phospho-ERK were inhibited by compound 106 in both the sensitive and resistant cell lines in a concentration dependent manner. Importantly, protein levels of Cyclin D1, which phosphorylates retinoblastoma protein (pRB) were decreased in parallel with a decrease in phospho-pRB protein in sensitive cell lines. Protein levels of p27, an inhibitor of CDK, were increased in the presence of compound 106 in the sensitive line. These results demonstrate that zearalenone analog compounds transcriptionally inhibit Cyclin D1 expression followed by inhibition of phosphorylation of pRB protein, leading to cell cycle arrest. Thus, these markers can serve as pharmacodynamic markers for response to treatment with a zearalenone analog compound, *e.g.*, Compound 106.

**Example 10: Immunohistochemistry of Human Melanoma Tissues**

Formalin-fixed, paraffin-embedded human melanoma tumor samples were obtained and processed for immunohistochemistry (IHC) with the antibodies described in Table 10. IHC procedure (1) described below was used if the incubation with primary antibody was for 1 hour as indicated in Table 10. IHC procedure (2) described below was used if the incubation with primary antibody was overnight as indicated in Table 10.

**Table 10:** Antibodies, Retrievals and Incubation Conditions for Immunohistochemistry

| **Ab name** | **Source, Cat#** | **Species Type** | **Retrieval (96°C, 20 min)** | **Primary Ab, final conc** | **Primary Ab, incubation** | **Localization** |
|---|---|---|---|---|---|---|
| ERK 1/2 | Cell Signaling ® Cat. No. 4695 | Rabbit Polyclonal IgG[1] | Citrate pH 6 | 0.44 $\mu$g/mL | Overnight at 4 °C | cytoplasm |
| p-ERK 1/2 Thr202/Tyr 204 | Sigma®, Cat. No. M9692 | Mouse Monoclonal IgG1[2] | EDTA pH 8.0 | 3 $\mu$g/mL | 1 hr, Rm temp | nuclear |
| AKT(pan specific) | Cell Signaling ® Cat. No. 4691 | Rabbit Polyclonal IgG[3] | Citrate pH 6 | 0.5 $\mu$g/mL | Overnight at 4 °C | cytoplasm |
| Cyclin D1 | Lab Vision, Cat. No RB-9041 | Rabbit, Epitope Specific IgG[4] | Citrate pH 6 | 2 $\mu$g/mL | 1 hr, Rm temp | nuclear |

(continued)

| Ab name | Source, Cat# | Species Type | Retrieval (96°C, 20 min) | Primary Ab, final conc | Primary Ab, incubation | Localization |
|---|---|---|---|---|---|---|
| p-AKT (Ser473) | Cell Signaling ®Cat.No. 3787 | Rabbit Polyclonal IgG[5] | Citrate pH 6 | 0.5 μg/mL | Overnight at 4 °C | cytoplasm |
| p-RB (Ser780) | Sigma®, Cat. No. R6275 | Rabbit Polyclonal IgG[6] | EDTA pH 8.0 | 06 μg/mL | 1 hr, Rm temp | nuclear |
| RB | Cell Signaling ®Cat. No. 9309 | Mouse Monoclonal IgG2a[7] | Citrate pH 6 | 14 μg/mL | 1 hr, Rm temp | nuclear |
| Ki67 | Lab Vision, Cat. No. RB-9043 | Rabbit, Epitope Specific IgG | Citrate pH 6 | 067 μg/mL | 1 hr. Rm temp | nuclear |

[1] Rabbit mAb 137F5 detects endogenous levels of total p44/42 MAP kinase (ERK1/ERK2) protein
[2] Monoclonal anti-MAP kinase antibody reacts specifically with the diphosphorylated form of MAP kinase (ERK-1 and ERK-2)
[3] AKT (pan) (C67E7) rabbit mAb detects endogenous levels of total AKT protein
[4] This rabbit mAb detects a C-terminal epitope of Cyclin D1.
[5] Rabbit mAb 736E11 detects AKT1 if phosphorylated at serine 473 and detects AKT2 and AKT3 if phosphorylated at the corresponding site
[6] Anti-phospho-Retinoblastoma (pSer780) antibody recognizes RB phosphorylated at Ser780 and does not react with non phosphorylated RB
[7] Mouse anti-RB mAb 4H1 detects endogenous levels of total RB protein

**a. IHC Procedure (1)**

IHC Procedure (1) was used for all Primary Antibodies requiring a 1-hour incubation at Room Temperature. IHC methods were conducted using Lab Vision Autostainer 360 and Lab Vision LP-AP detection kits (Ultra Vision LP Large Volume Detection System AP Polymer (Ready-To-Use), Catalog No. TL-125-AL). Human melanoma sections (5 μ thickness) were deparafinized and rehydrated. Epitope retrieval was conducted in Lab Vision Pretreatment Module, 96°C, no boiling cycle, 20 mintues, followed by a 20 minute cooling period (to 75°C). Retrieval buffers were either EDTA or Citrate, as indicated in Table 10: (a) EDTA buffer, pH 8.0 (Lab Vision, Cat. No. TA-250-PM2X); or (b) Citrate buffer, pH 6.0 (Lab Vision, Cat. No. TA-250-PM1X).

Slides were transferred to the Autostainer and the following program was used: Pre-rinse (TBS-Tween buffer, Lab Vision, Cat. No. TA-999-TT); Non-specific protein block, 10 min (UV Block, part of the LP-AP kit); Primary Antibody: 60 min; 3 rinses in TBS-Tween Buffer; Antibody Enhancer (part of the LP-AP kit),10 min; 3 rinses in TBS-Tween Buffer; Labeled Polymer - AP conjugated (part of the LP-AP kit), 15 min; 3 rinses in TBS-Tween Buffer; Substrate: Fast Red (prepared from the Fast Red substrate system (Lab Vision, Cat. No. TA-125-AF)); 1 rinse in water; and 2 rinses in distilled water.

Slides were counterstained with hematoxylin Gill III (VWR®, Cat. No. 15204-268). (This chromogen is not solvent resistant, and cannot be used with xylene / alcohols). The hematoxylin counterstain procedure was as follows: Hematoxylin, 30 sec; $dH_2O$; Tap water rinse, 5 min; Bluing reagent, 30 sec; Tap water rinse, 5 min; $dH_2O$.

Slides were cover-slipped manually using Vision Mount Mounting Media (Lab Vision, Cat. No. TA-125-UG). This mounting medium is compatible with Lab Vision's Fast Red. Number 1.5 coverslip glass was used for optimal microscope analysis (with objectives optimized for No. 1.5 coverslips). Analyses were done within 1 week of IHC staining, because Lab Vision Fast Red is not permanent.

**b. IHC Procedure (2)**

IHC Procedure (2) was used for all Primary Antibodies requiring an overnight (18 hr) incubation at 4 °C.

THC methods were conducted using Lab Vision Autostainer 360 and Lab Vision LP-AP detection kits (UltraVision LP Large Volume Detection System AP Polymer (Ready-To-Use), Cat. No. TL-125-AL). Human melanoma sections (5 μ thickness) were deparafinized and rehydrated. Epitope retrieval was conducted in Lab Vision Pretreatment Module, 96 °C, no boiling cycle, 20 min, followed by a 20 min cooling period (to 75 °C). Retrieval buffers were either EDTA or

Citrate, as indicated in Table 10: (a) EDTA buffer, pH 8.0 (Lab Vision, Cat. No. TA-250-PM2X); or (b) Citrate buffer, pH 6.0 (Lab Vision, Cat. No. TA-250-PM1X).

The steps up to and including the primary Ab were done manually: Pre-rinse (TBS-Tween buffer, Lab Vision, Cat. No. TA-999-TT); Non-specific protein block, 10 min (UV Block, part of the LP-AP kit); Primary Antibody: overnight (18 hours) at 4 °C in the humidity chamber (to prevent the slides from drying out). On overnight incubations, a hydrophobic barrier pen was used to draw an oval around the tissue to prevent the Ab from running off the slide during the long incubation period.

Slides were then transferred to the Autostainer and the following program was used: 3 rinses in TBS-Tween Buffer; Antibody Enhancer (part of the LP-AP kit), 10 min; 3 rinses in TBS-Tween Buffer; Labeled Polymer - AP conjugated (part of the LP-AP kit), 15 min; 3 rinses in TBS-Tween Buffer; Substrate: Fast Red (prepared from the Fast Red substrate system, Lab Vision, Cat. No. TA-125-AF); 1 rinse in water; and 2 rinses in distilled water.

Slides were counterstained with hematoxylin Gill III (VWR Cat. No. 15204-268). (This chromogen is not solvent resistant, and cannot be used with xylene / alcohols). The hematoxylin counterstain procedure was as follows: Hematoxylin, 30 sec; $dH_2O$; Tap water rinse, 5 min; Bluing reagent, 30 sec; Tap water rinse, 5 min; $dH_2O$.

Slides were coverslipped manually using Vision Mount Mounting Media (Lab Vision, Cat. No. TA-125-UG). Number 1.5 coverslip glass was used for optimal microscope analysis (with objectives optimized for No. 1.5 coverslips). Analyses were done within 1 week of IHC staining, because Lab Vision Fast Red is not permanent.

The foregoing procedures and conditions were effective in detecting ERK 1/2, phospho-ERK, AKT, Cyclin D1, p-AKT, p-RB, RB and Ki67 in human melanoma tumor samples (commercially available clinical samples).

## Example 11: Time-dependent Inhibition of ERK and Phospho-pRb Phosphorylation in LOX Xenografts

LOX human melanoma cells, which carry the V600E BRAF mutation, were originally obtained from the DCTD Tumor Repository (Frederick, MD). The cells were grown in monolayer cultures in DCTD-recommended growth media at 37 °C in a 5% $CO_2$ humidified incubator. On the day of subcutaneous (s.c.) injections of the cells, growth medium was removed, flasks were washed with PBS, and cells were collected with trypsinization. The cells were washed and collected by centrifugation, and then resuspended in ice-cold PBS at a concentration of $1x10^7$ cells/mL. The cells ($1x10^6$ cells) were inoculated s.c. in female athymic NU/NU mice near the right axillary area using a 27-gauge needle with a volume of 0.1 mL, and allowed to grow. Compound 106 was administered intravenously (i.v.) on a single dosing at the dosage level of 40 mg/kg with a volume of injection 0.1 mL per 10g body weight. Then, the mice were euthanized at different time points including 0, 1, 2, 4, 8, 12, 24, 48, 72 h after dosing. The tumor tissue was dissected and fixed with 10% formalin for immunohistochemistry.

For immunohistochemical detection of phospho-ERK 1/2, rabbit mAb 20G11 was used at a final concentration of 0.36 μg/ml (Cell Signaling, Cat. No. 4376). This mAb detects endogenous levels of p44 and p42 MAP Kinase (ERK1 and ERK2) when dually phosphorylated at Thr202 and Tyr204 of ERK1 (Thr185 and Tyr187 of ERK2), and singly phosphorylated at Thr202. Conditions were essentially as described in IHC Procedure (2), with incubation overnight (18 hr) at 4 °C and EDTA retrieval buffer. Secondary reagent was goat anti-rabbit antibody, conjugated to alkaline phosphatase.

Results are shown in Figure 9A. Phospho-ERK staining was observed at the 0 hour time-point. Phospho-ERK staining steadily declined until essentially no observable staining was evident by the 8 hour time-point. By 12 hours, phospho-ERK staining was again observed, and essentially recovered to baseline levels within about 24 hours. Ki67 staining, which detects a marker of cell proliferation, decreased substantially over the first 12 hours and was suppressed to below detection between 24 and 48 hours, through 72 hours.

These results demonstrate that phospho-ERK can be used as a pharmacodynamic marker to monitor the efficacy of treatment with a zearalenone analog compound, and decreased levels are indicative that a cancer is sensitive to treatment.

For immunohistochemical detection of phospho-RB, anti-phospho-Retinoblastoma (pSer780) antibody (Sigma, Cat. No. R6275) was used (Table 10). Conditions were as described in IHC Procedure (1), with incubation for one hour at room temperature and EDTA retrieval buffer. Secondary reagent was anti-rabbit antibody, conjugated to horseradish peroxidase.

Results are shown in Figure 9B. Prominent phospho-pRB staining was observed initially; however, phospho-pRB level was suppressed to below detection between 48 and 72 hours. These results demonstrate that phospho-pRB can be used as a pharmacodynamic marker to monitor the efficacy of treatment with a zearalenone analog compound, and that decreased levels of phospho-pRB indicate that a cancer is sensitive to treatment with a zearalenone analog compound.

## Example 12: Effects of Compound 106 Treatment on Growth of Subcutaneous DBTRG-05MG Human Glioblas-

**toma Xenografts *In Vivo***

The purpose of this study was to investigate anticancer activity of Compound 106 in a human DBTRG-05MG glioblastoma xenograft model. DBTRG-05MG human glioblastoma cancer cells, which carry the *BRAF* (V600E) mutation, were grown in monolayer cultures in ATCC-recommended growth media at 37 °C in a 5% $CO_2$ humidified incubator. Cells were expanded in T225 flasks for the *in vivo* study. On the day of injection, growth medium was removed, flasks were washed with PBS, and cells were collected by trypsinization. The cells were washed and collected by centrifugation, and resuspended in ice-cold PBS.

Female athymic NU/NU mice (6 week old, Charles River Laboratories (Wilmington, MA)) were inoculated subcutaneously (s.c.) with $5x10^6$DBTRG-05MG human glioblastoma cancer cells. Those animals that developed tumors of approximately 150 $mm^3$ were selected and randomized into five groups.

The study consisted of a vehicle-treated group and four drug-treated groups of 8 mice per group for a total of 40 mice on the first day of treatment. All treatments were initiated on day 21. Compound 106 was administered intravenously (i.v.) on a Q4D x 3 (days 21, 25, and 29) dosing schedule at dosages of 5, 10, 20 and 40 mg/kg with a volume of injection of 0.1 mL per 10 g body weight (Q4D x 3 = every four days for a total of three injections). The control group was treated with vehicle alone (20% Captisol in water; Captisol® (Sulfobutyl Ether Beta Cycolodextrin, Sodium Salt; Cydex, Inc., KS)).

General health of the mice was monitored and mortality was recorded daily. Tumor dimensions and animal body weights were recorded twice a week starting on the first day of treatment. The s.c. tumor volumes were measured and animals were weighed twice weekly starting with the first day of treatment. Tumor volume was determined by caliper measurement (mm) and using the following formula:

$$(l \text{ x } w^2)/2 = mm^3$$

where l and w refer to the larger and smaller dimensions obtained from each measurement.

Relative body weight (RBW) was calculated as follows:

RBW = body weight on the day of measurement/body weight on the first day of treatment.

The mean and standard error of the mean (SEM) of tumor volume and relative body weight for each experimental group were calculated.

The study was terminated 60 days after cancer cell transplantation. In each Compound 106 treatment group, measurements were also terminated when average tumor volume reached two doublings (4-fold tumor volume) from the first day of treatment.

Statistical analysis of the control group versus Compound 106 treatment groups was performed by a one way analysis of variance (ANOVA) followed by Dunnett's multiple comparison test for each dose of test compound in the experiment for tumor volume. A value of $P < 0.05$ was considered statistically significant under a two-sided hypothesis. All statistical analyses were performed using GraphPad Prism® software (version 4, San Diego, CA). The results are shown in Figure 10. Data show the mean + SEM. Animals were treated intravenously on days 21, 25, and 29 (Q4D x 3; indicated by arrows in the figure). Asterisks (*) indicate $P < 0.01$ vs. control group.

Administration of Compound 106 at all four doses tested, 5, 10, 20, and 40 mg/kg, caused statistically significant anticancer activity. One out of eight animals in each of the groups treated with Compound 106 at dosages of 5 mg/kg and 20 mg/kg, respectively, were tumor-free on day 60. These results indicate that growth of s.c.-implanted DBTRG-05MG human glioblastoma xenografts (which carry the *BRAF* (V600E) mutation) was highly sensitive to intermittent administration of Compound 106 at all dosages tested, 5, 10, 20, and 40 mg/kg.

**Example 13. Effects of Compound 106 Treatment on Growth of Subcutaneous LOX Human Melanoma Xenografts *In Vivo***

The purpose of this study was to investigate anticancer activity of Compound 106 in a LOX human melanoma xenograft model. LOX human melanoma cells, which carry a *BRAF* (V600E) mutation, were obtained from the DCTD Tumor Repository (Frederick, MD). Cells were grown in monolayer cultures in T225 flask with RPMI-1640 growth media at 37 °C in a 5% $CO_2$ humidified incubator. Cells were further cultivated in T225 flasks for the in vivo study. On the day of subcutaneous (s.c.) injections of the cells, growth medium was removed, flasks were washed with PBS, and cells were collected with trypsinization. Cells were washed and centrifuged for 3 minutes, and then resuspended in ice-cold PBS.

Female athymic NU/NU mice were inoculated s.c. with 1 x$10^6$ LOX human melanoma cells. Those animals that developed tumors of approximately 150 $mm^3$ were selected and randomized to five groups. The study consisted of a vehicle-treated group and four drug-treated groups of 8 mice per group for a total of 40 mice on the first day of treatment. All treatments were initiated on day 6. Compound 106 was administered intravenously (i.v.) on a Q4Dx3 (days 6, 10, and 14) dosing schedule at dosages of 5, 10, 20 and 40 mg/kg with a volume of injection of 0.1 mL per 10 g body weight. The control group was treated with vehicle (20% Captisol in water) alone.

The s.c. tumor volumes were measured and animals were weighed twice weekly starting with the first day of treatment. Tumor volume and relative body weight (RBW) were determined as described in Example 12.

The study was terminated fifty-nine days after cancer cell transplantation. For each Compound 106 treatment group, measurements were also terminated when mean tumor volume reached two doublings (4-fold tumor volume) from the first day of treatment.

Statistical analysis was by ANOVA as described in Example 12, and the results are shown in Figure 11. Data show the mean + SEM. Animals were treated intravenously on days 6, 10, and 14 (Q4D x 3; indicated by arrows in Figure 11). Tumor measurements on tumor bearing mice in all surviving groups were stopped on day 37. Then, tumor measurements were continued for tumor-free mice in the 10 mg/kg (1/8), 20 mg/kg (5/8), and 40 mg/kg (7/8) Compound 106 treatment groups. The study was terminated on day 59. Asterisks (*) indicate P<0.05 vs. control group.

Administration of Compound 106 at three of the doses tested, 10, 20, and 40 mg/kg, caused statistically significant anticancer activity. Additionally, one, five, and seven out of eight animals in the groups treated with Compound 106 at doses of 10, 20, or 40 mg/kg, respectively, were tumor-free at the end of the study on day 59. These results indicate that growth of s.c.-implanted LOX human melanoma xenografts (which carry the *BRAF* (V600E) mutation and are wild-type for *PTEN*) was highly sensitive to intermittent administration of Compound 106 at doses of 10, 20, and 40 mg/kg.

Compound 106 was also tested in other xenograft models, in which *BRAF*-mutated cells, including breast, colon, and melanoma cell lines, or wildtype cells, including pancreatic cell lines, were transplanted subcutaneously into female nude mice. Compound 106 or a vehicle control was administered intravenously. In the *BRAF*-mutated xenograft models, at 40 mg/kg of Compound 106 and a dosing regimen of QD x 5 for 2 weeks, Compound 106 showed inhibitory effects ranging from tumor stasis (but not regression) during treatment (> 80% tumor growth inhibition) to about 73% tumor regression. In the wildtype xenograft models, at 40 mg/kg of Compound 106 and a dosing regimen of QD x 5 for 2 weeks, Compound 106 showed inhibitory effects ranging from 30-40% tumor regression.

The application includes the following clauses:

39. A method of prognosing the ability of a zearalenone analog compound of formula I to treat a cance*r in a subject, the method comprising determining whether a sample derived from said subject exhibits a mutation in the *BRAF* gene which increases, BRAF activity, wherein the presence of a mutation in the *BRAF* gene in said sample as compared to a control sample indicates that a zearalenone analog compound has the ability to treat the cancer in the subject, thereby prognosing the ability of a zearalenone analog compound to treat the cancer in the subject.

40. The method of claim 39, wherein the mutation in the *BRAF* gene is V600E.

41. The method of claim 39, wherein the mutation in the *BRAF* gene is a mutation in the kinase domain of BRAF.

42. The method of claim 39, wherein the mutation in the *BRAF* gene is selected from the group consisting of V600E, G464E, G464V, G466A, G466E, G466V, G469A, G469E, E586K, F595L, G596R, L597V, L597R, L597S and V600D.

43. The method of claim 39, wherein determining whether said sample exhibits a mutation in the *BRAF* gene is accomplished using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of said sample.

44. The method of claim 39, wherein determining whether said sample exhibits a mutation in the *BRAF* gene comprises measuring BRAF activity in said sample, wherein an increase in BRAF activity in said sample as compared to the control sample is an indication of a mutation in the *BRAF* gene.

## Claims

**1.** A method of prognosing the ability of a zearalenone analog compound to treat a cancer in a subject, the method

comprising determining whether a sample derived from said subject exhibits a mutation in the *BRAF* gene which increases BRAF activity, wherein the zearalenone analog compound is a compound of formula (I)

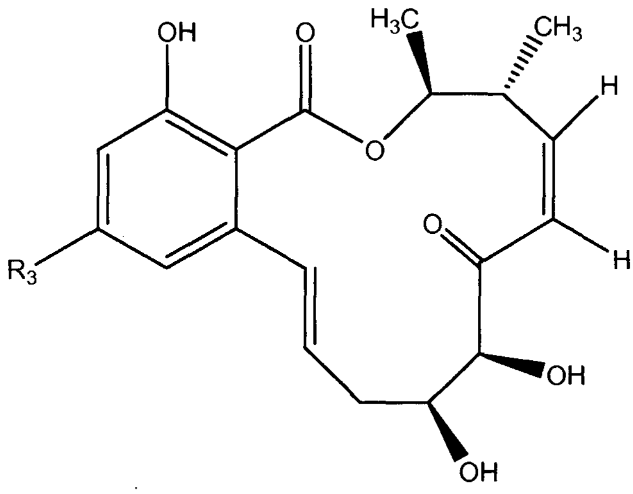

(I)

wherein $R_3$ is $-NHR_1$, and $R_1$ is $C_1$-$C_3$ alkyl substituted with 0, 1, or 2 hydroxyl moieties, or a pharmaceutically acceptable salt or ester thereof, and
wherein the presence of a mutation in the *BRAF* gene in said sample as compared to a control sample indicates that a zearalenone analog compound has the ability to treat the cancer in the subject, thereby prognosing the ability of a zearalenone analog compound to treat the cancer in the subject.

**2.** The method of claim 1, wherein the mutation in the BRAF gene is V600E or wherein the mutation in the BRAF gene is a mutation in the kinase domain of BRAF.

**3.** The method of claim 1, wherein the mutation in the BRAF gene is selected from the group consisting of V600E, G464E, G464V, G466A, G466E, G466V, G469A, G469E, E586K, F595L, G596R, L597V, L597R, L597S and V600D.

**4.** The method of claim 1, wherein determining whether said sample exhibits a mutation in the BRAF gene is accomplished using a technique selected from the group consisting of polymerase chain reaction (PCR) amplification reaction, reverse-transcriptase PCR analysis, single-strand conformation polymorphism analysis (SSCP), mismatch cleavage detection, heteroduplex analysis, Southern blot analysis, Western blot analysis, and deoxyribonucleic acid sequencing of said sample.

**5.** The method of claim 1, wherein determining whether said sample exhibits a mutation in the BRAF gene comprises measuring BRAF activity in said sample, wherein an increase in BRAF activity in said sample as compared to the control sample is an indication of a mutation in the BRAF gene.

**6.** The method of any one of claims 1-5, wherein the zearalenone analog compound is a compound of formula (I) or a pharmaceutically acceptable salt or ester thereof, and wherein $R_3$ is an unsubstituted $C_{1-3}$ alkyl-amino or wherein $R_3$ is a group selected from the group consisting of methylamino and ethylamino.

**7.** The method of claim 6, wherein the zearalenone analog compound is the compound:

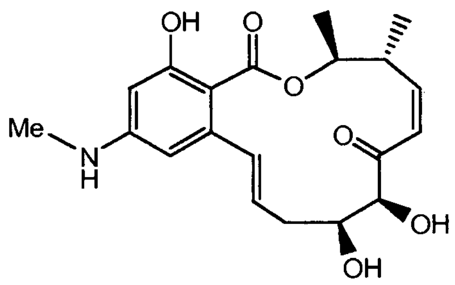

(Compound 091)

or a pharmaceutically acceptable salt or ester thereof or wherein the zearalenone analog compound is

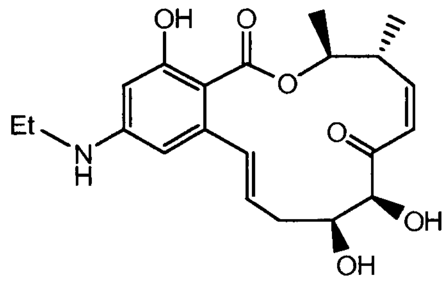

(Compound 106)

or a pharmaceutically acceptable salt or ester thereof.

**Patentansprüche**

1. Verfahren zum Prognostizieren der Fähigkeit einer Zearalenon-Analogverbindung, Krebs bei einem Individuum zu behandeln, wobei das Verfahren umfasst: Bestimmen, ob eine von diesem Individuum stammende Probe eine Mutation in dem *BRAF*-Gen aufweist, die die BRAF-Aktivität erhöht, wobei die Zearalenon-Analogverbindung eine Verbindung der Formel (I) ist:

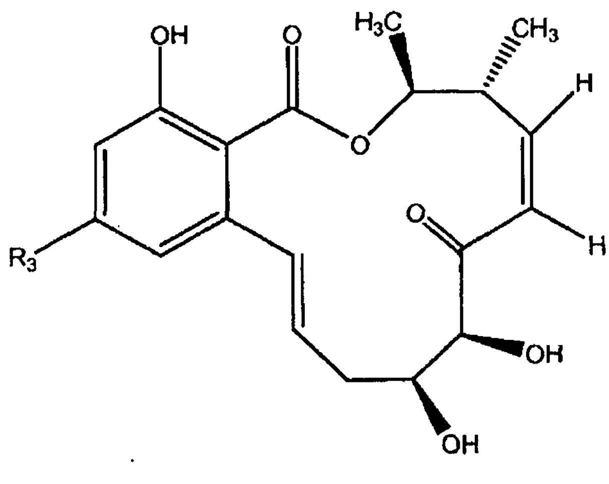

(I)

worin $R_3$ -$NHR_1$ ist und $R_1$ $C_1$-$C_3$-Alkyl, substituiert mit 0, 1 oder 2 Hydroxyl-Moietäten, ist, oder ein pharmazeutisch akzeptables Salz oder Ester davon, und wobei das Vorhandensein einer Mutation in dem BRAF-Gen in dieser Probe im Vergleich zu einer Kontrollprobe anzeigt, dass eine Zearalenon-Analogverbindung die Fähigkeit aufweist, Krebs

bei dem Individuum zu behandeln, dadurch Prognostizieren der Fähigkeit einer Zearalenon-Analogverbindung, Krebs bei dem Individuum zu behandeln.

2. Verfahren gemäß Anspruch 1, wobei die Mutation in dem BRAF-Gen V600E ist oder wobei die Mutation in dem BRAF-Gen eine Mutation in der Kinase-Domäne von BRAF ist.

3. Verfahren gemäß Anspruch 1, wobei die Mutation in dem BRAF-Gen ausgewählt ist aus der Gruppe, bestehend aus V600E, G464E, G464V, G466A, G466E, G466V, G469A, G469E, E586K, F595L, G596R, L597V, L597R, L597S und V600D.

4. Verfahren gemäß Anspruch 1, wobei das Bestimmen, ob diese Probe eine Mutation in dem BRAF-Gen aufweist, unter Anwendung einer Technik erreicht wird, die ausgewählt ist aus der Gruppe, bestehend aus Amplifikationsreaktion der Polymerase-Kettenreaktion (PCR), Reverse Transkriptase-PCR-Analyse, Einzelstrang-Konformations-Polymorphismus-Analyse (SSCP), Fehlpaarungsspaltungsdetektion, Heteroduplexanalyse, Southern-Blot-Analyse, Western-Blot-Analyse und Desoxyribonukleinsäuresequenzierung der Probe.

5. Verfahren gemäß Anspruch 1, wobei das Bestimmen, ob die Probe eine Mutation im BRAF-Gen aufweist, das Messen der BRAF-Aktivität in der Probe umfasst, wobei ein Anstieg der BRAF-Aktivität in der Probe im Vergleich zu der Kontrollprobe ein Hinweis auf eine Mutation im BRAF-Gen ist.

6. Verfahren gemäß irgendeinem der Ansprüche 1-5, wobei die Zearalenon-Analogverbindung eine Verbindung der Formel (I) oder ein pharmazeutisch akzeptables Salz oder Ester davon ist, und worin $R_3$ ein unsubstituiertes $C_{1-3}$-Alkylamino ist oder worin $R_3$ eine Gruppe ist, ausgewählt aus der Gruppe, bestehend aus Methylamino und Ethylamino.

7. Verfahren gemäß Anspruch 6, wobei die Zearalenon-Analogverbindung die Verbindung:

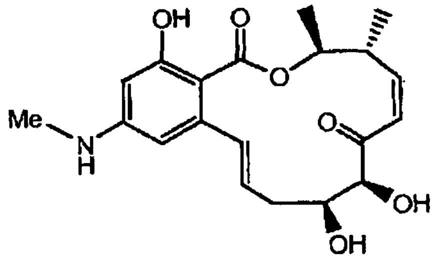

(Verbindung 091)

oder ein pharmazeutisch akzeptables Salz oder Ester davon ist, oder wobei die Zearalenon-Analogverbindung

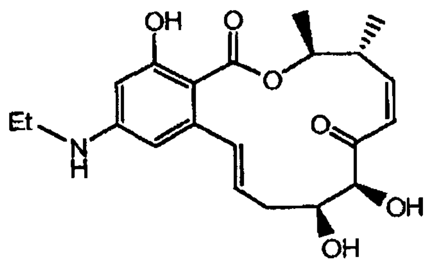

(Verbindung 106)

oder ein pharmazeutisch akzeptables Salz oder Ester davon ist.

**Revendications**

1. Procédé de pronostic de la capacité d'un composé analogue de la zéaralénone à traiter un cancer chez un sujet,

le procédé comprenant la détermination si un échantillon dérivé dudit sujet présente une mutation dans le gène BRAF qui augmente l'activité de BRAF, le composé analogue de la zéaralénone étant un composé de formule (I)

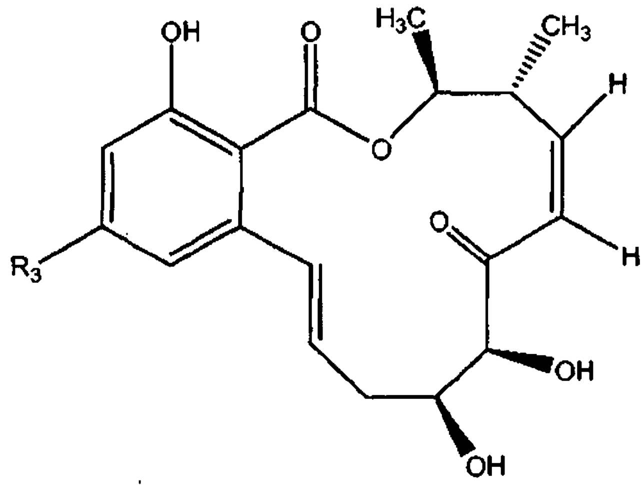

(I)

dans laquelle $R_3$ est -$NHR_1$, et $R_1$ est un alkyle en $C_1$ à $C_3$ substitué avec 0, 1 ou 2 fragments hydroxyle, ou un sel ou ester acceptable sur le plan pharmaceutique de celui-ci, et où la présence d'une mutation dans le gène BRAF dans ledit échantillon par comparaison avec un échantillon témoin indique qu'un composé analogue de la zéaralénone a la capacité de traiter le cancer chez le sujet, pronostiquant de ce fait la capacité d'un composé analogue de la zéaralénone à traiter le cancer chez le sujet.

2. Procédé selon la revendication 1, dans lequel la mutation dans le gène BRAF est V600E ou dans lequel la mutation dans le gène BRAF est une mutation dans le domaine de kinase de BRAF.

3. Procédé selon la revendication 1, dans lequel la mutation dans le gène BRAF est choisie dans le groupe constitué de V600E, G464E, G464V, G466A, G466E, G466V, G469A, G469E, E586K, F595L, G596R, L597V, L597R, L597S et V600D.

4. Procédé selon la revendication 1, dans lequel la détermination si ledit échantillon présente une mutation dans le gène BRAF est accomplie en utilisant une technique choisie dans le groupe constitué d'une réaction d'amplification en chaîne par polymérase (PCR), une analyse par PCR à transcriptase inverse, une analyse de polymorphisme de conformation des acides nucléiques simple brin (SSCP), une détection de clivage à mésappariement, une analyse d'hétéroduplex, une analyse par buvardage de Southern, une analyse par buvardage de Western, et un séquençage d'acide désoxyribonucléique dudit échantillon.

5. Procédé selon la revendication 1, dans lequel la détermination si ledit échantillon présente une mutation dans le gène BRAF comprend la mesure de l'activité de BRAF dans ledit échantillon, dans lequel une augmentation de l'activité de BRAF dans ledit échantillon par comparaison avec l'échantillon témoin est une indication d'une mutation dans le gène BRAF.

6. Procédé selon l'une quelconque des revendications 1 à 5, dans lequel le composé analogue de la zéaralénone est un composé de formule (I) ou un sel ou ester acceptable sur le plan pharmaceutique de celui-ci, et dans lequel $R_3$ est un (alkyl en $C_{1-3}$)-amino ou dans lequel $R_3$ est un groupe choisi parmi le groupe constitué de méthylamino et éthylamino.

7. Procédé selon la revendication 6, dans lequel le composé analogue de la zéaralénone est le composé :

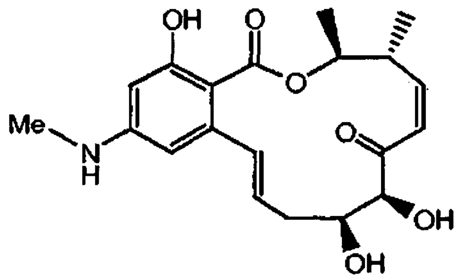

(Composé 091)

ou un sel ou ester acceptable sur le plan pharmaceutique de celui-ci ou dans lequel le composé analogue de la zéaralénone est

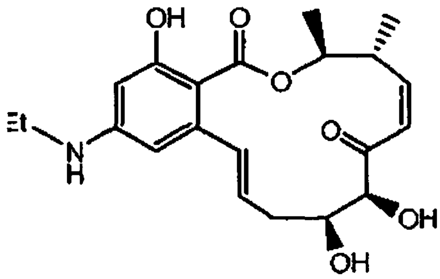

(Composé 106)

ou un sel ou ester acceptable sur le plan pharmaceutique de celui-ci.

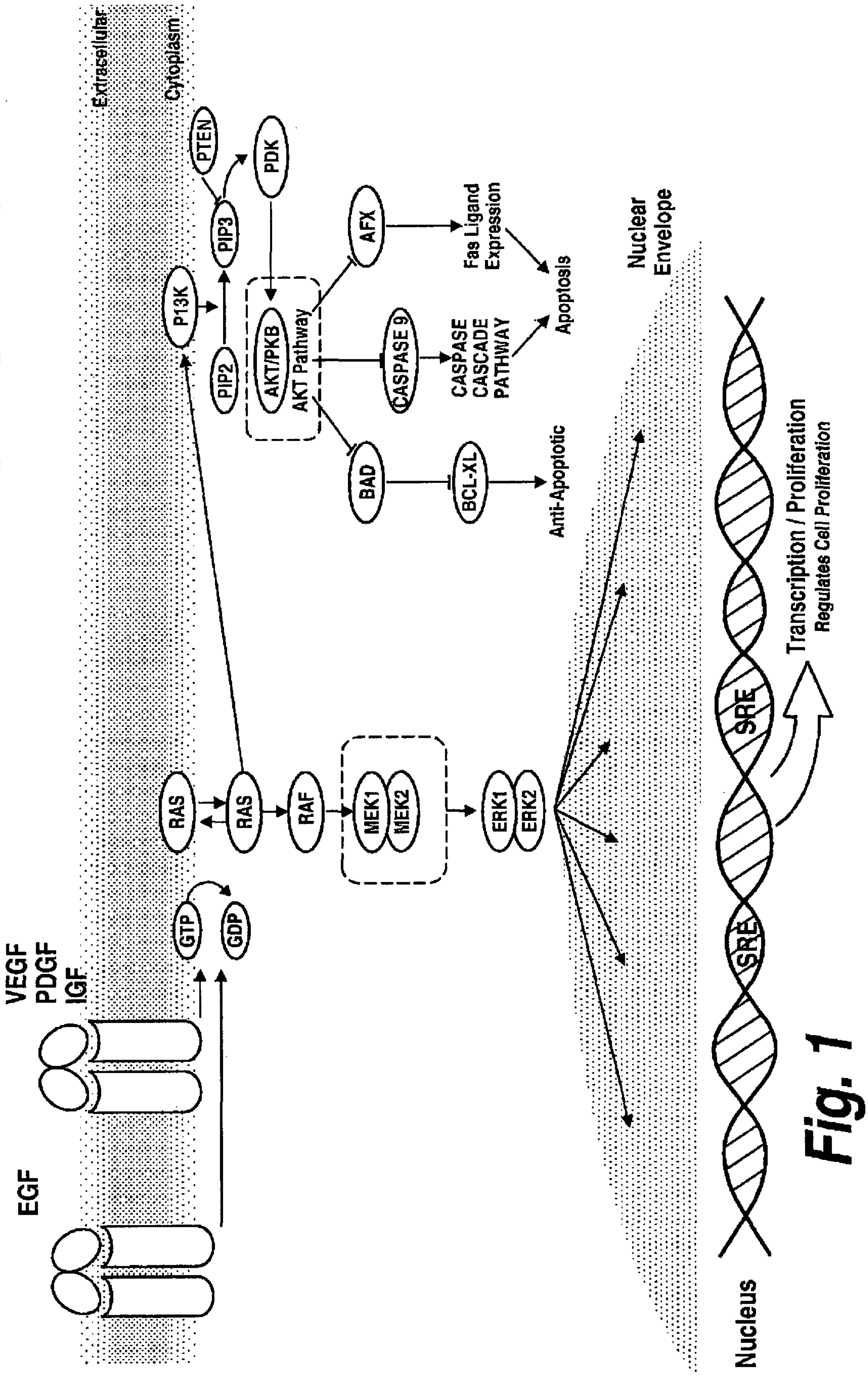
The RAS/RAF/MEK/ERK MAPK and PI3K Signaling Pathways
EGF
VEGF
PDGF
IGF
Extracellular
Cytoplasm
GTP
GDP
RAS
RAS
RAF
MEK1
MEK2
ERK1
ERK2
P13K
PIP2
PIP3
PTEN
PDK
AKT/PKB
AKT Pathway
BAD
BCL-XL
Anti-Apoptotic
CASPASE 9
CASPASE
CASCADE
PATHWAY
AFX
Fas Ligand
Expression
Apoptosis
Nuclear
Envelope
Nucleus
SRE
SRE
Transcription / Proliferation
Regulates Cell Proliferation

Fig. 1

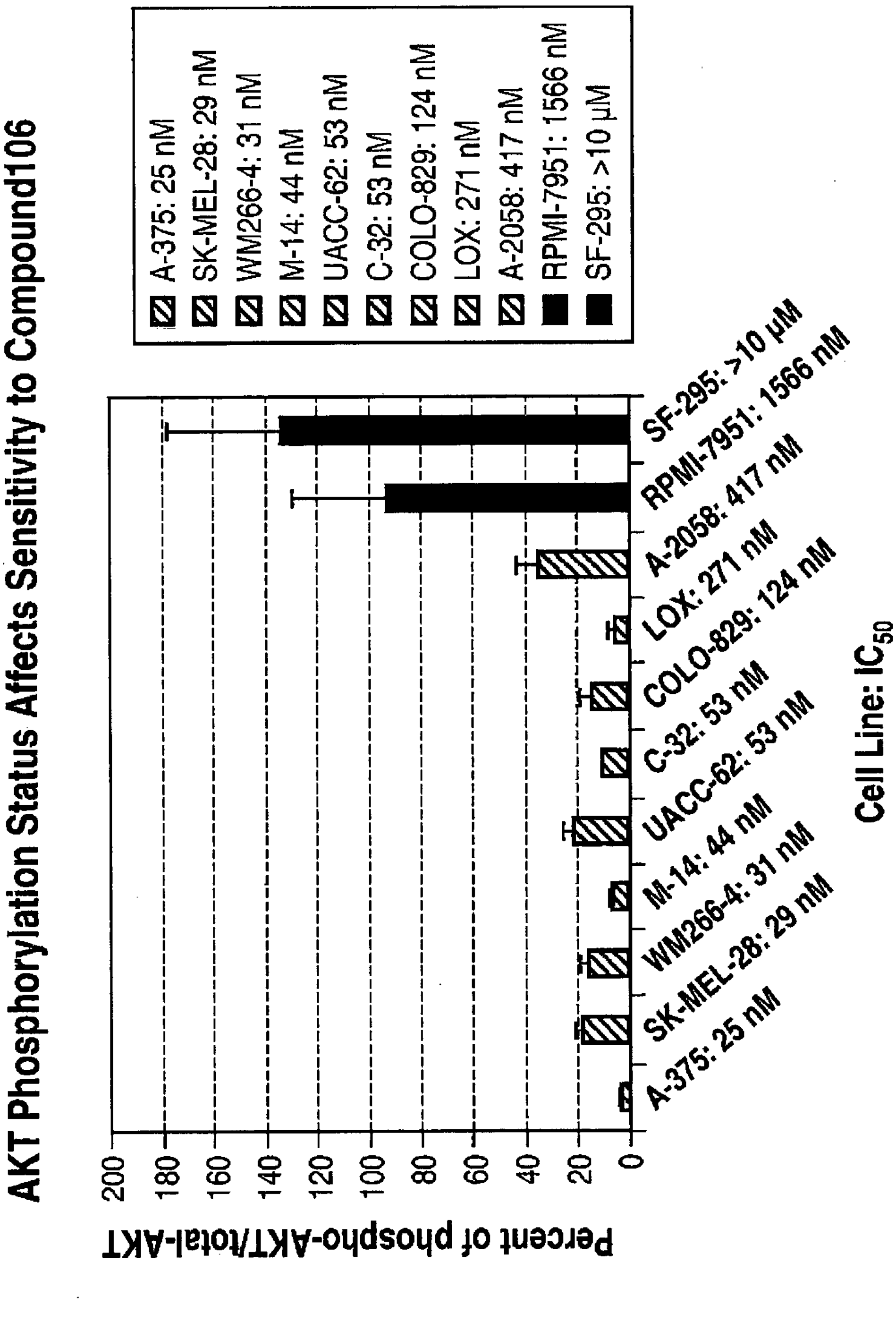
AKT Phosphorylation Status Affects Sensitivity to Compound106
Percent of phospho-AKT/total-AKT
200
180
160
140
120
100
80
60
40
20
0
A-375: 25 nM
SK-MEL-28: 29 nM
WM266-4: 31 nM
M-14: 44 nM
UACC-62: 53 nM
C-32: 53 nM
COLO-829: 124 nM
LOX: 271 nM
A-2058: 417 nM
RPMI-7951: 1566 nM
SF-295: >10 μM
Cell Line: IC50

Fig. 2

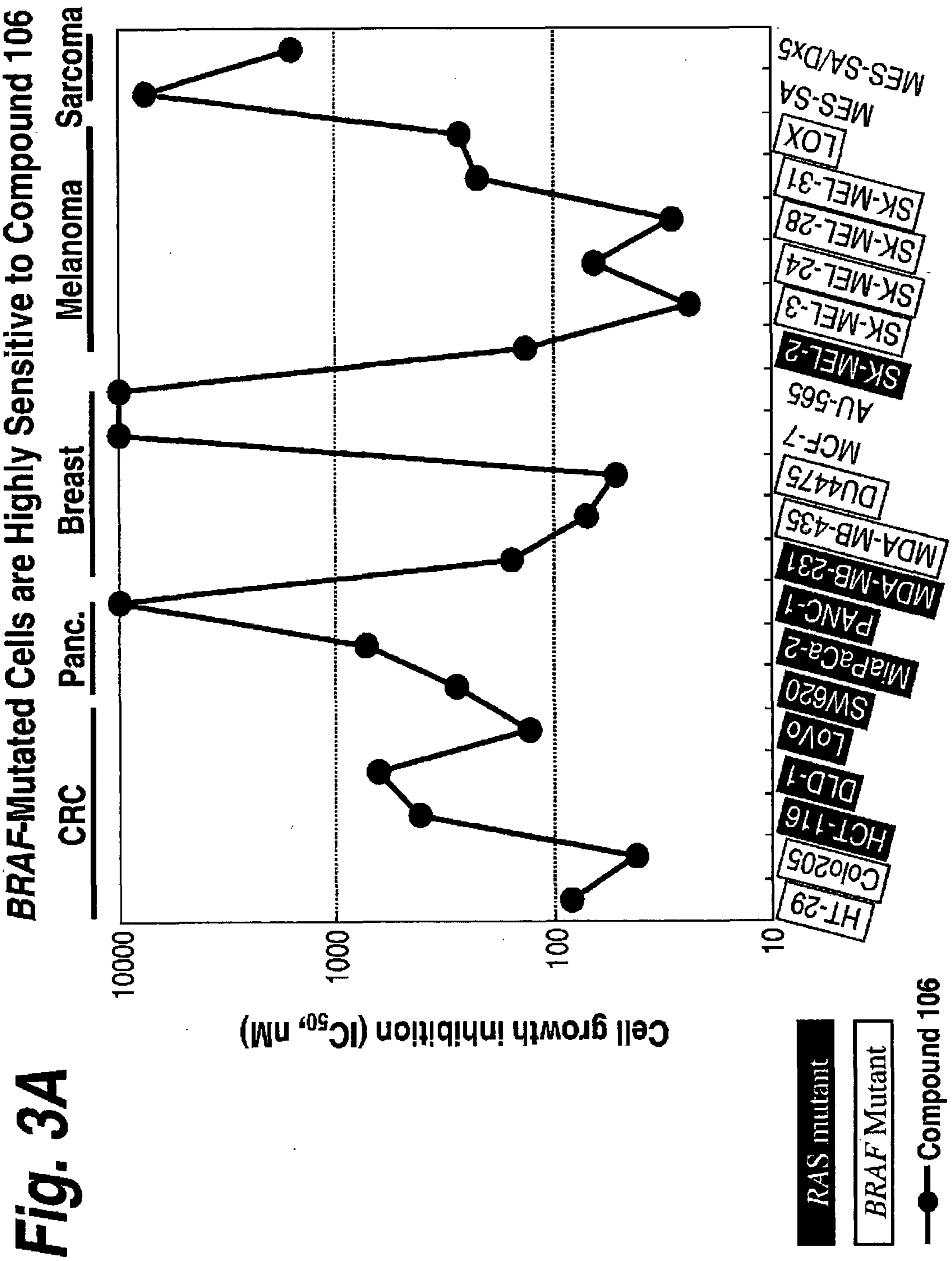
Fig. 3A
BRAF-Mutated Cells are Highly Sensitive to Compound 106
CRC
Panc.
Breast
Melanoma
Sarcoma
Cell growth inhibition ($IC_{50}$, nM)
10000
1000
100
10
HT-29
Colo205
HCT-116
DLD-1
LoVo
SW620
MiaPaCa-2
PANC-1
MDA-MB-231
MDA-MB-435
DU4475
MCF-7
AU-565
SK-MEL-2
SK-MEL-3
SK-MEL-24
SK-MEL-28
SK-MEL-31
LOX
MES-SA
MES-SA/Dx5
RAS mutant
BRAF Mutant
Compound 106

## Inhibition of *BRAF* Mutated Cancer Cell Growth

| Cell Line | Description | *K-RAS* mutation | *BRAF* mutation | Mean $IC_{50}$, nmol/L | |
|---|---|---|---|---|---|
| | | | | Cpd 106 | Cpd 091 |
| AU-565 | breast cancer | | WT | >10000 | >10000 |
| MCF-7 | | | WT | >10000 | >10000 |
| MDA-MB-231 | | G13D | WT | 156 | 119 |
| MDA-MB-435 | | | V600E | 71 | 50 |
| DU4475 | | | V600E | 52 | 30 |
| SK-MEL-2 | melanoma | Q61R (N-ras) | WT | 135 | 113 |
| SK-MEL-3 | | | V600E | 24 | 19 |
| SK-MEL-24 | | | V600E | 66 | 53 |
| SK-MEL-31 | | | V600E | 225 | 209 |
| HCT-116 | colon cancer | G13D | WT | 423 | 257 |
| DLD-1 | | G13D | WT | 649 | 466 |
| LoVo | | G13D | WT | 132 | 90 |
| SW-620 | | G12V | WT | 282 | 207 |
| COLO205 | | WT | V600E | 43 | 27 |
| HT-29 | | WT | V600E | 84 | 65 |
| Mia PaCa-2 | pancreatic cancer | G12C | WT | 734 | 582 |
| PANC-1 | | G12D | WT | >10000 | >10000 |
| BxPC-3 | | WT | WT | - | 412 |
| MES-SA | sarcoma | | WT | 7490 | 5780 |
| MES-SA/Dx5-Rx1 | sarcoma (P-glycoprotein overex.) | | WT | 1590 | 908 |
| IMR-90 | Non-dividing fibroblast | | | - | >10000 |

"WT" = wild type; "-" = not tested

***Fig. 3B***

## $IC_{50}$ Values for Compound 106 in a Panel of Melanoma Cell Lines

| Cell Line No. | $IC_{50}$ (nmol/L) | *BRAF* | Type (*BRAF*) | *PTEN* | pAKT |
|---|---|---|---|---|---|
| 1 | 7 | V600R | homo | wt | 0 |
| 2 | 8 | V600E | hetero | wt | 0 |
| 3 | 8 | V600E | hetero | wt | 0 |
| 4 | 13 | V600E | hetero | F56I | 2 |
| 5 | 21 | V600E | hetero | wt | 0 |
| 6 | 26 | V600E | hetero | wt | 0 |
| 7 | 29 | L597S | homo | wt | 1 |
| 8 | 46 | V600E | hetero | wt | 1 |
| 9 | 46 | V600E | hetero | wt | 3 |
| 10 | 53 | V600E | hetero | wt | 0 |
| 11 | 64 | V600E | hetero | L139X | 2 |
| 12 | 66 | V600E | hetero | wt | 0 |
| 13 | 68 | V600E | hetero | Q298X | 1 |
| 14 | 69 | V600E | hetero | wt | 0 |
| 15 | 75 | V600E | hetero | wt | 2 |
| 16 | 201 | V600E | hetero | Y76X | 1 |
| 17 | 318 | V600E | hetero | wt | 0 |
| 18 | 894 | V600E | hetero | Splice Mutation | 3 |
| 19 | 1081 | V600E | hetero | HomoDeln | 3 |
| 20 | 3378 | L597S | homo | wt | 0 |
| 21 | 24 | wt | wt | wt | 0 |
| 22 | 43 | wt | wt | wt | 0 |
| 23 | 84 | wt | wt | wt | 0 |
| 24 | 120 | wt | wt | wt | 0 |
| 25 | 138 | wt | wt | wt | 0 |
| 26 | 201 | wt | wt | wt | 0 |
| 27 | 396 | wt | wt | wt | 0 |
| 28 | 520 | wt | wt | wt | 1 |
| 29 | 612 | wt | wt | wt | 3 |
| 30 | 739 | wt | wt | HomoDeln | 0 |
| 31 | 1669 | wt | wt | I50N | 1 |

***Fig. 3C***

## $IC_{50}$ Values for Compound 106 in a Panel of Cell Lines

| Cell Line | $IC_{50}$ (nmol/L) | BRAF | PTEN |
|---|---|---|---|
| Malme-3M | 13 | V600E | wt |
| A375 | 25 | V600E | wt |
| SK-MEL-28 | 28 | V600E | wt |
| WM-266-4 | 31 | V600E | wt |
| SH-4 | 40 | V600E | wt |
| SK-MEL-3 | 40 | V600E | wt |
| M14 | 44 | V600E | wt |
| C32 | 53 | V600E | Del-FS |
| UACC-62 | 53 | V600E | wt |
| SK-MEL-24 | 80 | V600E | Del-FS |
| A101D | 85 | V600E | wt |
| Colo-829 | 124 | V600E | wt |
| G-361 | 133 | V600E | wt |
| HT-144 | 182 | V600E | Del-In-fr |
| LOX | 300 | V600E | wt |
| A2058 | 417 | V600E | L112Q, V175Fs 2 |
| RPMI-7951 | 1566 | V600E | Del-FS |
| SK-MEL-31 | 225 | V600E | wt |

*Fig. 3D*

## Prognostic and Response Biomarkers

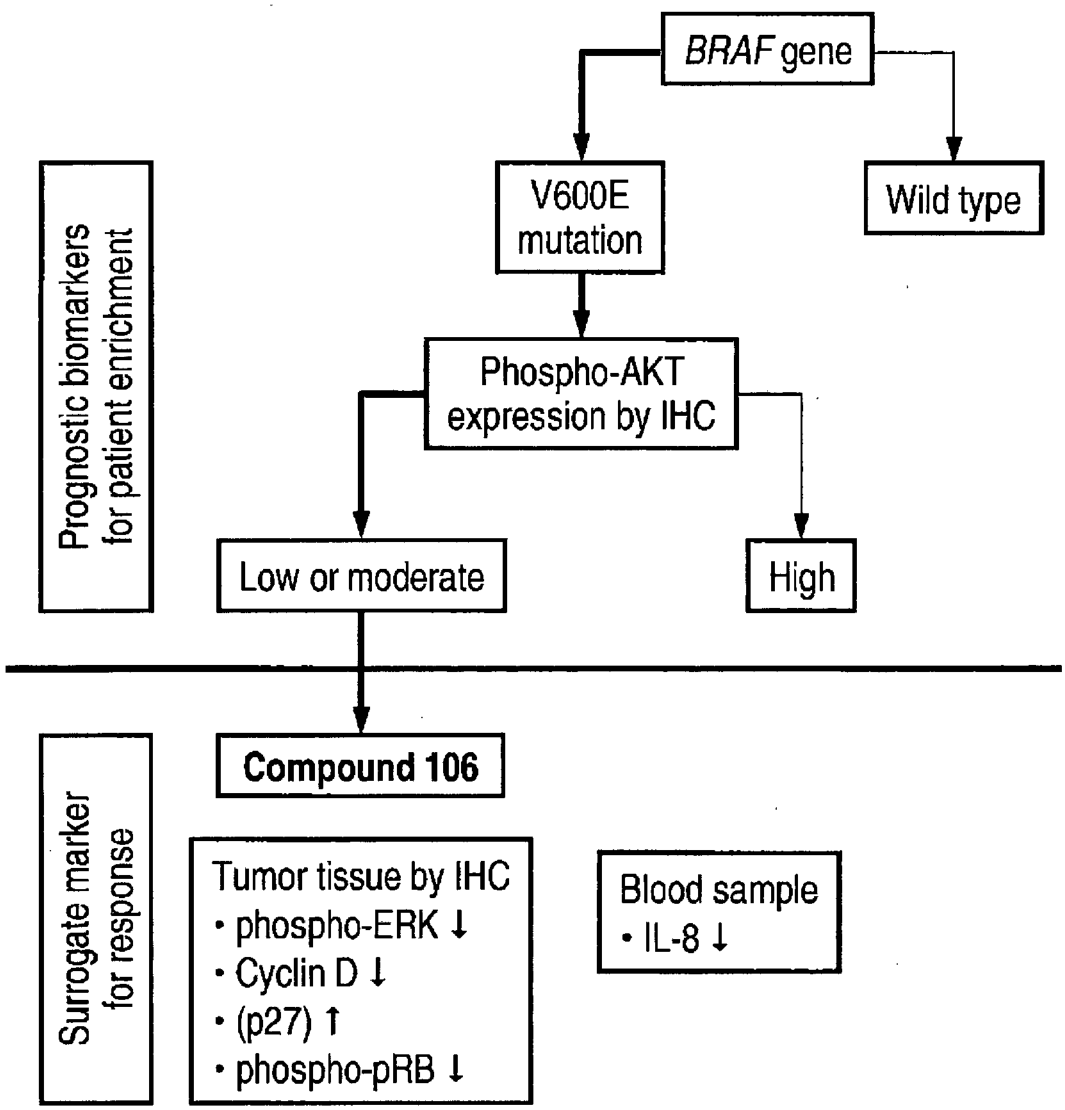


*Fig. 4*

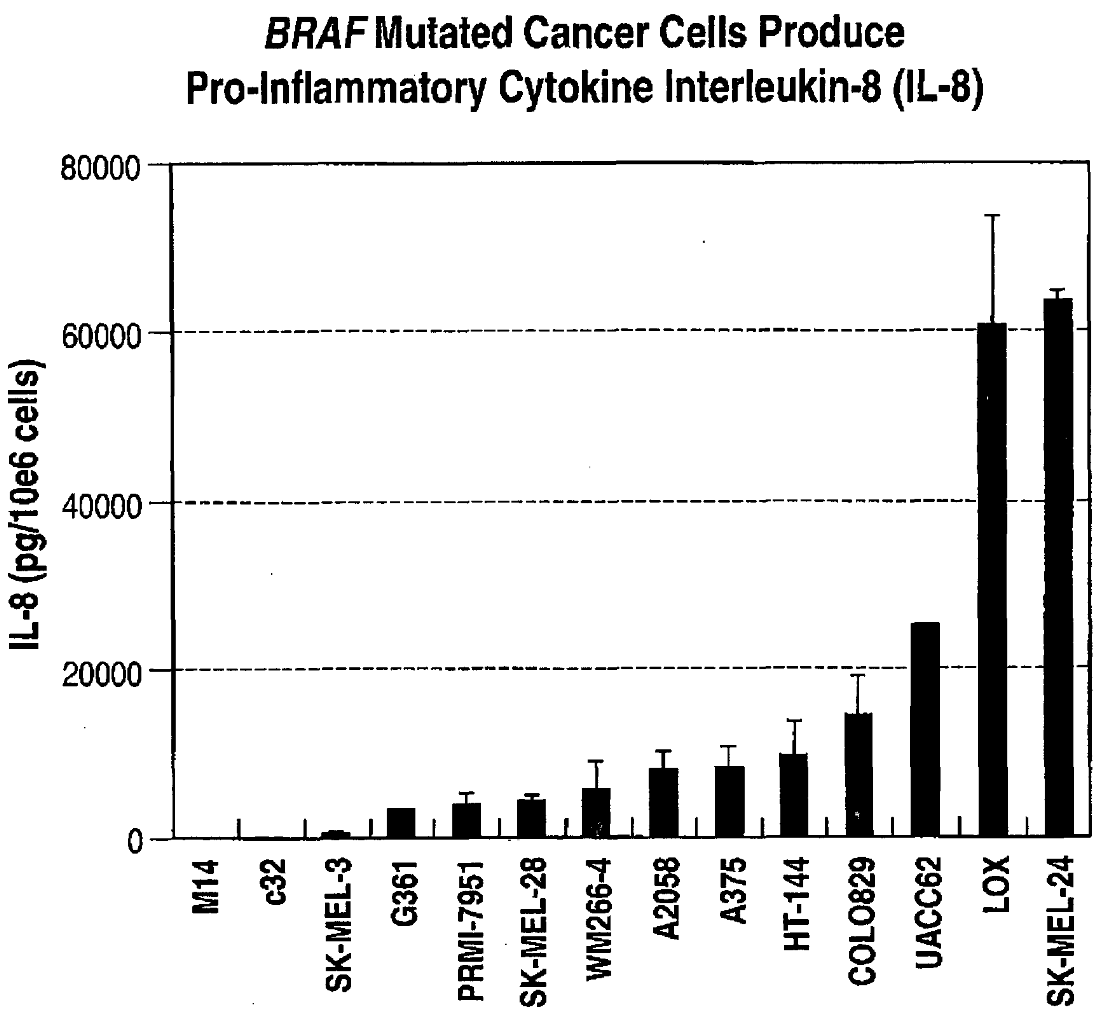
*BRAF* Mutated Cancer Cells Produce
Pro-Inflammatory Cytokine Interleukin-8 (IL-8)
IL-8 (pg/10e6 cells)
80000
60000
40000
20000
0
M14
c32
SK-MEL-3
G361
PRMI-7951
SK-MEL-28
WM266-4
A2058
A375
HT-144
COLO829
UACC62
LOX
SK-MEL-24

*Fig. 5*

IL-6 and IL-8 Protein Production is Inhibited by Compound 106 in LOX Melanoma Cells *in vitro*

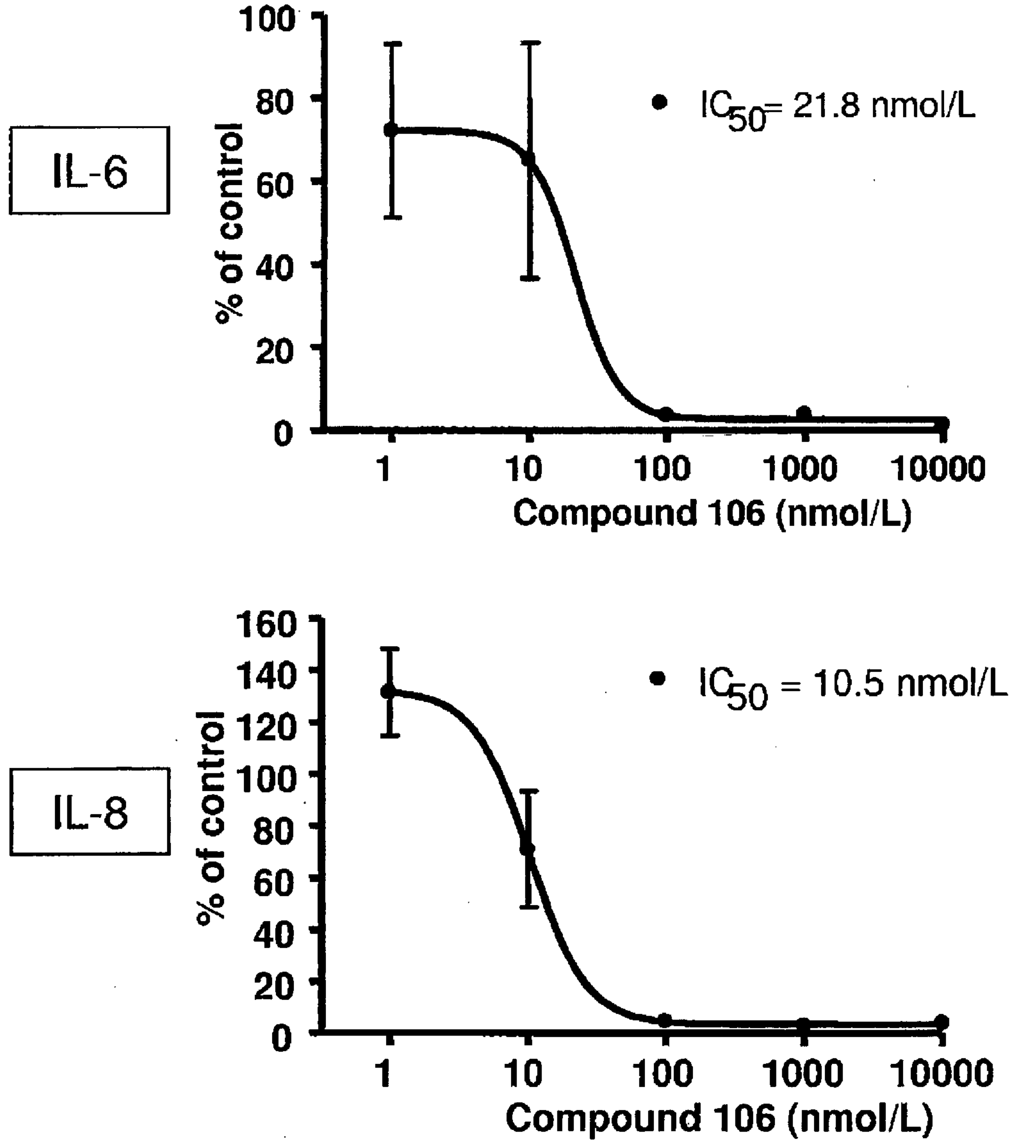


*Fig. 6*

The Change in Plasma IL-8 in Tumor Bearing Mice Treated with Compound 106

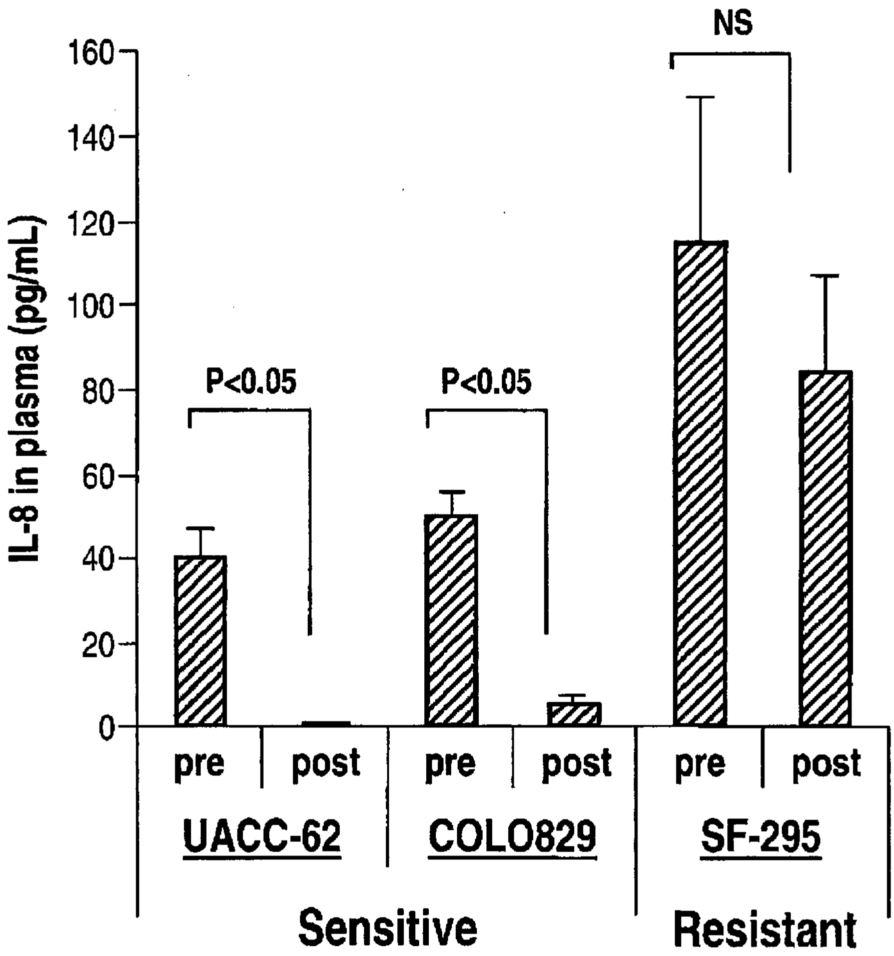


*Fig. 7*

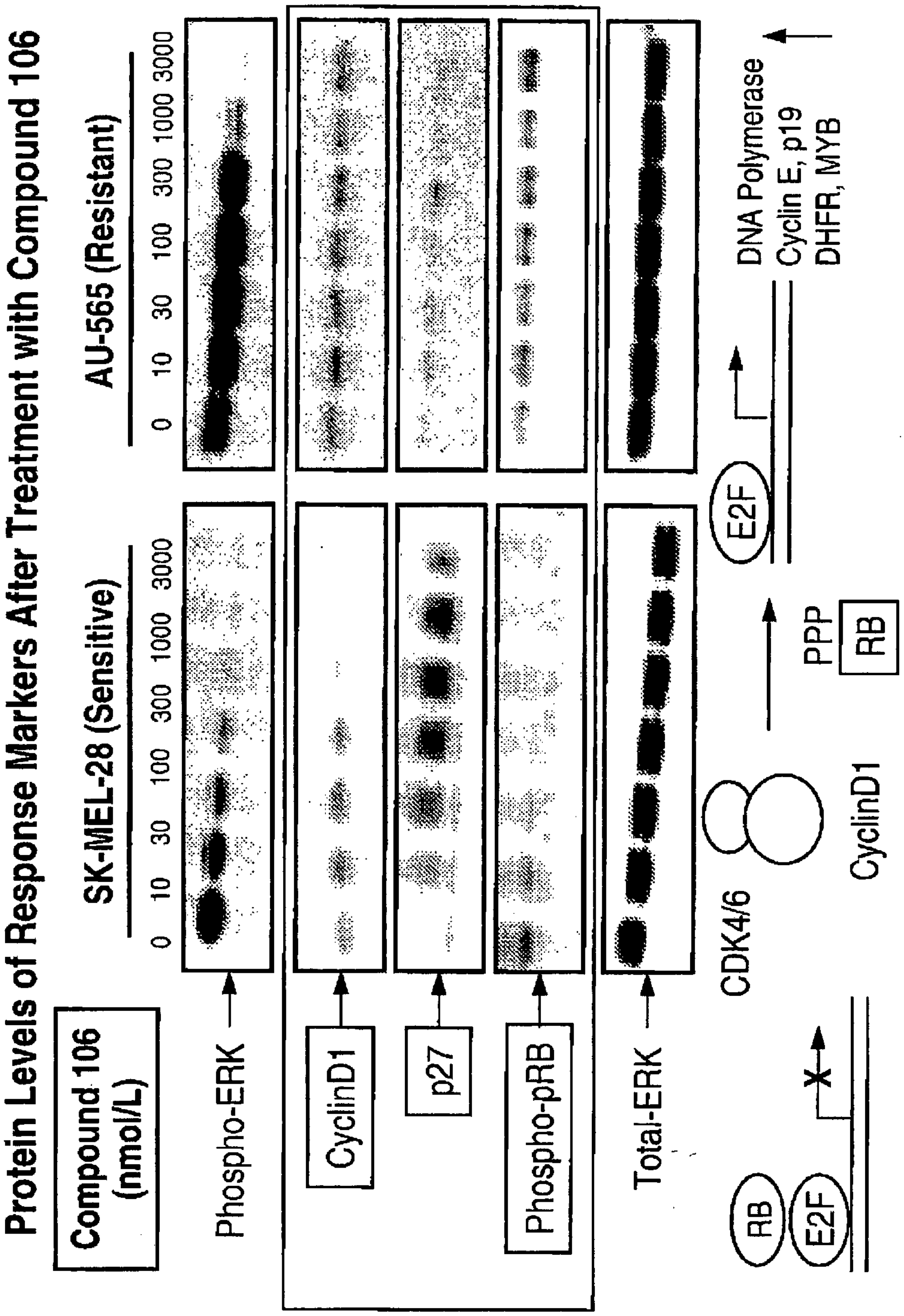

Protein Levels of Response Markers After Treatment with Compound 106
Compound 106 (nmol/L)
SK-MEL-28 (Sensitive)
AU-565 (Resistant)
0 10 30 100 300 1000 3000
0 10 30 100 300 1000 3000
Phospho-ERK
CyclinD1
p27
Phospho-pRB
Total-ERK
RB
E2F
CDK4/6
CyclinD1
PPP
RB
E2F
DNA Polymerase
Cyclin E, p19
DHFR, MYB


*Fig. 8*

phospho-ERK Staining

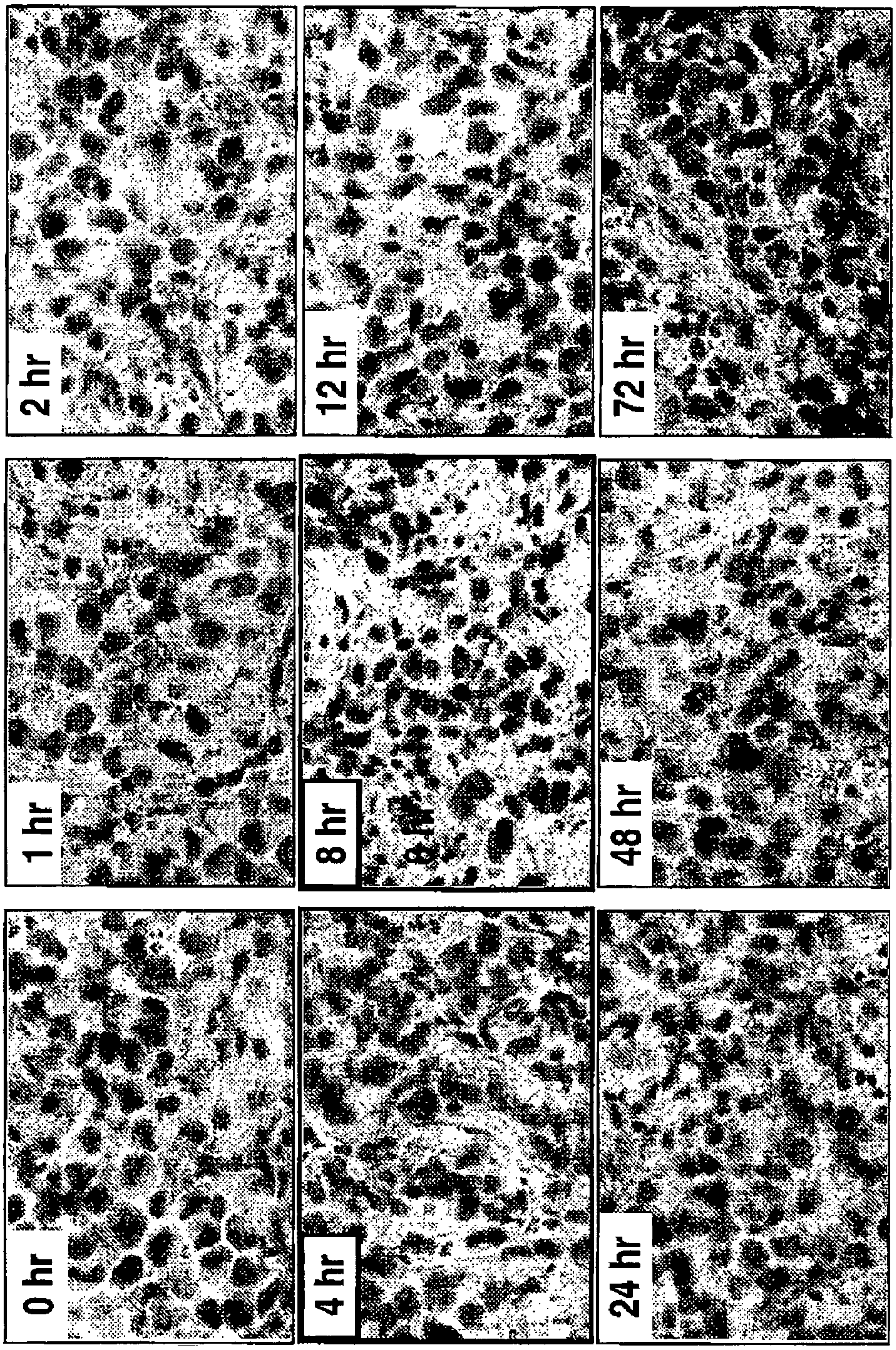


Fig. 9A

phospho-pRB Staining

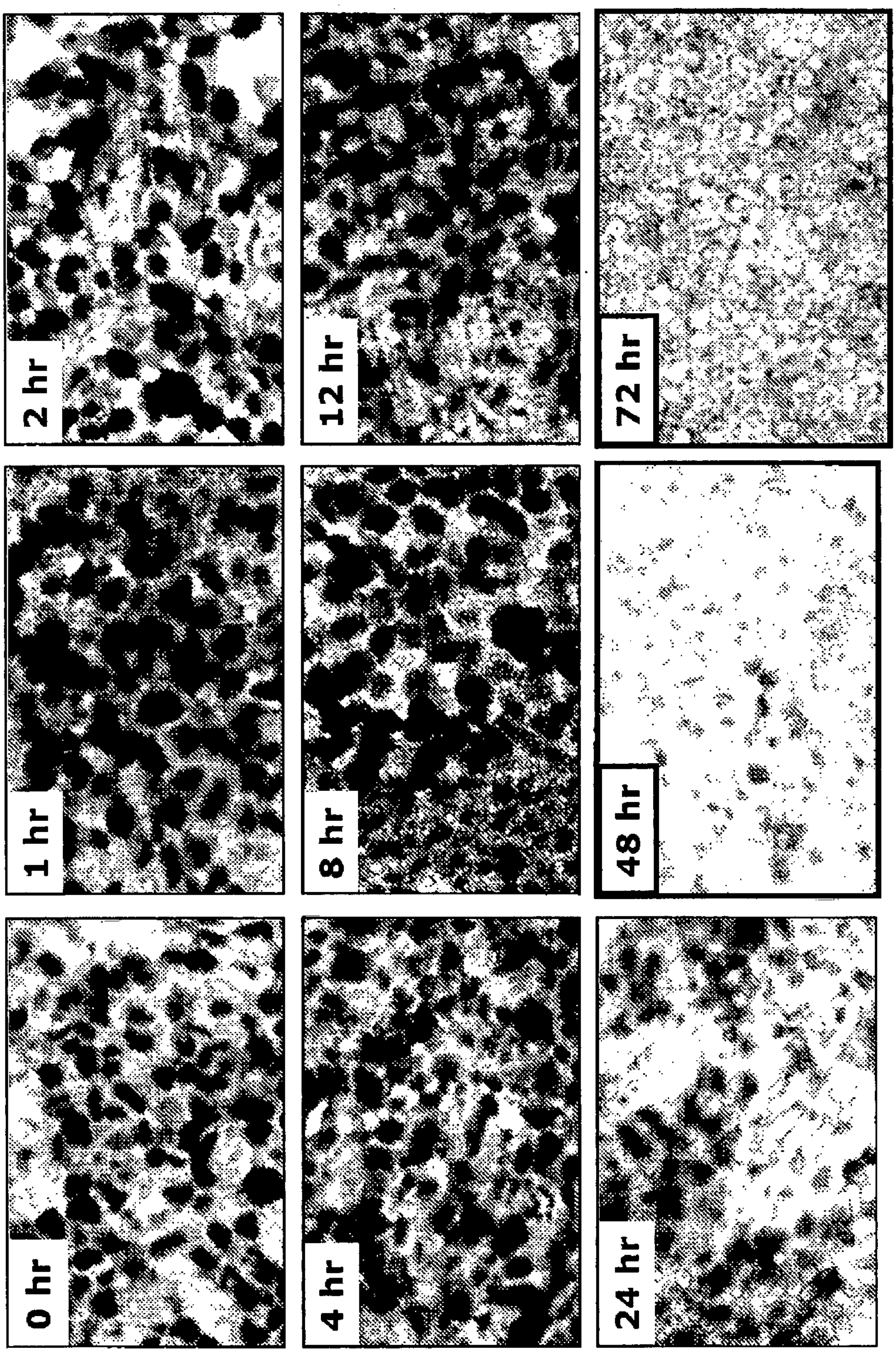


*Fig. 9B*

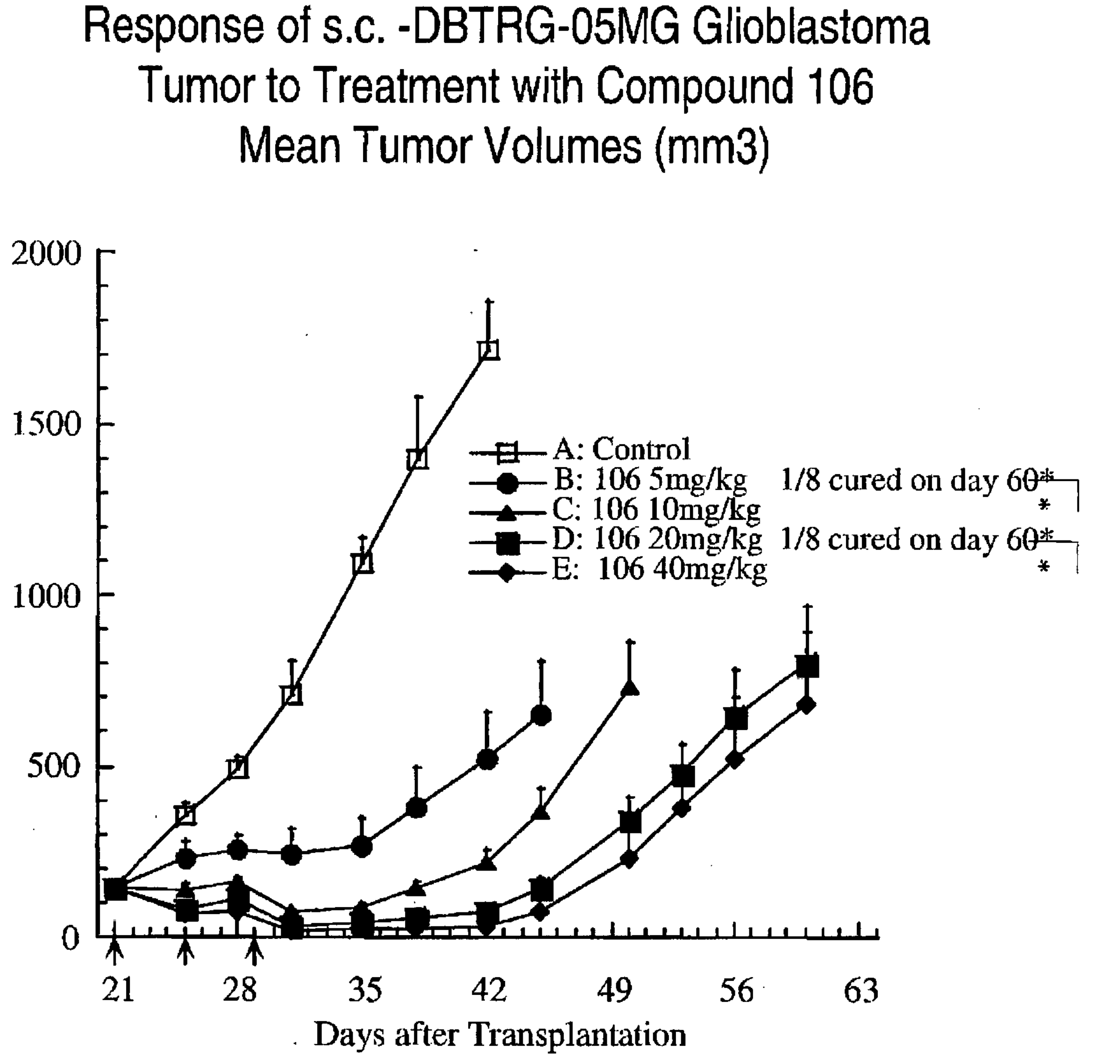
Response of s.c. -DBTRG-05MG Glioblastoma
Tumor to Treatment with Compound 106
Mean Tumor Volumes (mm3)
2000
1500
1000
500
0
21
28
35
42
49
56
63
Days after Transplantation
A: Control
B: 106 5mg/kg 1/8 cured on day 60*
C: 106 10mg/kg *
D: 106 20mg/kg 1/8 cured on day 60*
E: 106 40mg/kg *

*Fig. 10*

**Response of s.c.-LOX Melanoma Tumor to Treatment with Compound 106**

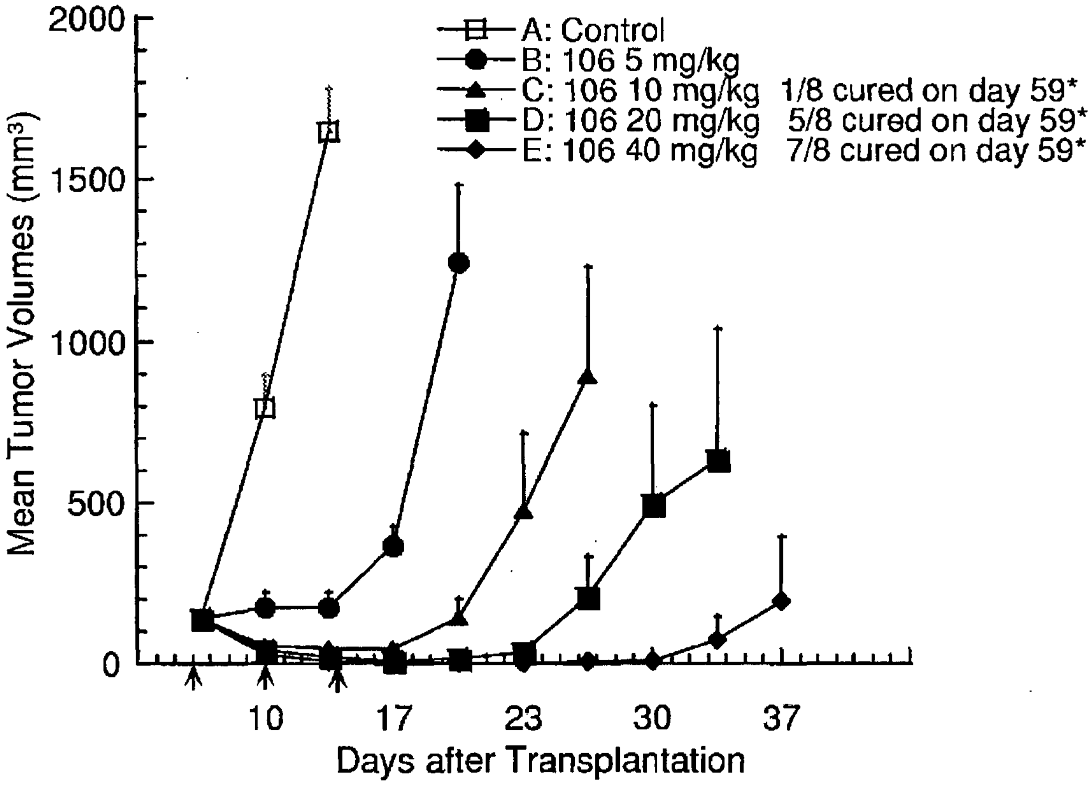


*Fig. 11*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60951906 B
- US 12180408 B
- WO 2006036941 A
- WO 9504136 A
- EP 0730740 B1
- US 5599681 A
- US 6942987 B
- US 6982318 B
- US 20020150954 A
- US 20070020232 A
- US 60951901 B
- US 60951892 B
- US 12180423 B
- US 507067 A
- US 20040224936 A
- GB 323845 A
- EP 606044 A
- WO 0038674 A
- JP 840893 A
- WO 9613259 A
- US 5728726 A
- US 5674892 A
- US 5795910 A
- WO 05023792 A
- WO 03076424 A


### Non-patent literature cited in the description


- **DAVIES et al.** *Nature,* 2002, vol. 417, 949-954
- **WALLACE et al.** *Current Topics in Medicinal Chemistry,* 2005, vol. 5, 215-219
- **SAMBROOK, J. et al.** Molecular Cloning: A Laboratory Manual. Cold Spring Harbor Laboratory Press, 1989
- **RODRIGUEZ-VICIANA et al.** *Science,* 2006, vol. 311, 1287-1290
- **DAVIES et al.** *Nature,* 2002, vol. 417, 949
- *Nature Reviews of Cancer,* 2004, 4
- **STOKOE et al.** *Science,* 1994, vol. 264, 1463-1467
- **YOON et al.** *Am. J. Physiol. Renal Physiol.,* 2004, vol. 286, F417-F424
- **DAVIES H. et al.** Mutations of the BRAF gene in human cancer. *Nature,* 2002, vol. 417, 949-954